# EUROPÄISCHE PATENTANMELDUNG

(11) **EP 0 047 977 A2**
(43) Veröffentlichungstag der Anmeldung: **24.03.1982**
(21) Anmeldenummer: 81107100.0
(22) Anmeldetag: 09.09.1981
(51) Int. Cl.: C07D 501/46, A61K 31/545

(54) **Cephalosporinverbindungen, Verfahren zu ihrer Herstellung, sie enthaltende pharmazeutische Präparate und deren Verwendung**

(30) Priorität: 12.09.1980 CH 6881/80; 07.05.1981 CH 2964/81
(71) Anmelder: CIBA-GEIGY AG, 4002 Basel (CH)
(72) Erfinder: Müller, Beat, Dr., CH-4106 Therwil (CH); Csendes, Ivan, Dr., CH-4104 Oberwil (CH)
(74) Vertreter: Zumstein, Fritz, Dr.

(57) **Zusammenfassung**

7β-Aminothiadiazolylacetylamino-3-cephem-4-carbonsäure-Verbindungen der Formel 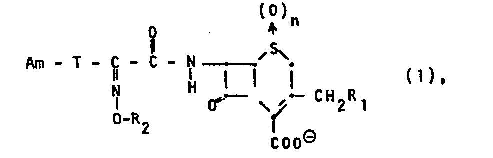 worin insbesondere Am Amino, T einen 1,2,4-Thiadiazolrest, R, einen gegebenenfalls substituierte Pyrazoliogruppe, eine gegebenenfalls substituierte Triazoliogruppe, eine Triniederalkylammoniogruppe oder eine substituierte Pyridiniogruppe und R₂ Wasserstoff, Niederalkyl, Carboxyniederalkyl, Carbamoyl oder N-substituiertes Carbamoyl bedeuten, besitzen antibiotische Eigenschaften und sind gegen grampositive und gramnegative Mikroorganismen wirksam. Die neuen Verbindungen werden in an sich bekannter Weise hergestellt. Ebenfalls umfasst sind Zwischenprodukte.

## Beschreibung

Die vorliegende Erfindung betrifft neue 7ß-Aminothiadiazolylacetyl- amino-3-ammoniomethyl-3-cephem-4-carbonsäureverbindungen, Verfahren zu ihrer Herstellung, pharmazeutische Präparate, welche solche Verbindungen enthalten, und ihre Verwendung zur Herstellung von pharmazeutischen Präparaten oder als pharmakologisch wirksame Verbindungen.

Die Erfindung betrifft insbesondere 7ß-Aminothiadiazolylacetyl- amino-3-ammoniomethyl-3-cephem-4-carbonsäureverbindungen der Formel 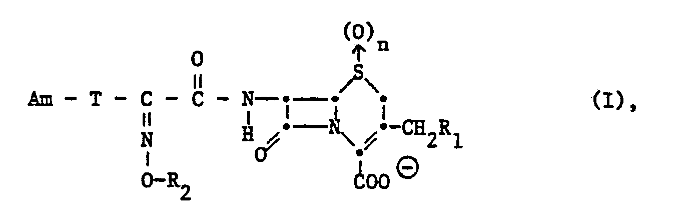 worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)- verbunden ist, R₁ eine unsubstituierte oder substituierte Pyrazoliogruppe, eine unsubstituierte oder substituierte Triazoliogruppe, eine Triniederalkylammoniogruppe oder eine Pyridiniogruppe der Formel

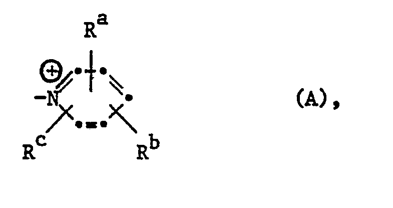

worin R^{a} durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl bedeutet, R Carbamoyl oder Wasserstoff bedeutet oder die Bedeutungen von R^{a} hat und R^{C} Wasserstoff bedeutet oder die Bedeutungen von R hat, und R₂ Wasserstoff, unsubstituiertes oder substituiertes Niederalkyl oder Cycloalkyl oder unsubstituiertes oder substituiertes Carbamoyl bedeuten, und Salze von solchen Verbindungen, die eine salzbildende Gruppe aufweisen, Verfahren zur Herstellung dieser Verbindungen, pharmazeutische Mittel, welche diese Verbindungen enthalten, und ihre Verwendung zur Herstellung von pharmazeutischen Präparaten oder als pharmakologisch wirksame Verbindungen.

In Verbindungen der Formel I hat der Index n in erster Linie den Wert 0. Falls n den Wert 1 hat, liegt das entsprechende 1-Oxid in der a- oder ß-Form vor.

In der vorliegenden Beschreibung bedeutet der im Zusammenhang mit Definition von Substituenten oder Verbindungen verwendete Ausdruck "nieder", dass die entsprechenden Substituenten oder Verbindungen, sofern nicht ausdrücklich anders definiert, bis zu 7, bevorzugt bis zu 4, Kohlenstoffatome enthalten.

Die vor- und nachstehend verwendeten Allgemeindefinitionen haben im Rahmen der vorliegenden Beschreibung vorzugsweise die folgenden Bedeutungen:
Substituiertes Amino Am ist beispielsweise durch die weiter unten beschriebenen Gruppen geschütztes Amino, d.h. leicht spaltbares und in freies Amino überführbares substituiertes Amino, insbesondere Acylamino. Es kann aber auch geschütztes sekundäres Amino oder tertiäres Amino sein, beispielsweise durch Niederalkyl mono-oder disubstituiertes Amino, insbesondere Methylamino.

Niederalkyl ist z.B. Methyl, Aethyl, n-Propyl, Isopropyl, n-Butyl, Isobutyl, sek.Butyl oder tert.-Butyl, ferner n-Pentyl, Neopentyl, n-Hexyl oder n-Heptyl.

Ein Thiadiazolrest T ist z.B. ein 1,2,4-Thiadiazolrest, der in 3- oder 5-Stellung mit Am, bzw. der Gruppe der Formel -C(=N-O-R₂)-, verbunden ist, wobei die Gruppe Am vorzugsweise in 5-Stellung mit dem 1,2,4-Thiadiazolrest T verknüpft ist. Weitere Thiadiazolreste T sind 1,2,3-Thiadiazolreste, welche in 4- und 5-Stellung mit der Gruppe Am bzw. der Gruppe der Formel -C(*N-0-R )-verbunden sind, sowie die symmetrischen 1,3,4- und 1,2,5-Thiadiazolreste, die jeweils an den beiden Ringkohlenstoffatomen mit der Gruppe Am bzw. der Gruppe der Formel -C(=N-O-R₂)- verbunden sind. Die durch eine Aminogruppe substituierte Thiadiazolgruppe T kann auch in tautomerer Form als eine durch eine Iminogruppe substituierte Dihydrothiadiazolgruppe oder als Gemisch der beiden Formen vorliegen. Das Gleichgewicht zwischen den beiden Tautomeren hängt von der Art der gegebenenfalls substituierten Aminogruppe Am, sowie von äusseren Faktoren, wie Temperatur, Lösungsmittel oder pH-Wert, ab. Im Rahmen der vorliegenden Erfindung wird die Thiadiazolgruppe in der Beschreibung und in den Ansprüchen nur als Thiadiazolgruppe bezeichnet, obgleich die Dihydrothiadiazolform ebenfalls umfasst ist.

Eine unsubstituierte oder substituierte Pyrazoliogruppe R₁ ist beispielsweise Pyrazolio oder durch Niederalkyl, z.B. Methyl, Aethyl, Propyl oder tert.-Butyl, Niederalkenyl, z.B. Vinyl oder Allyl, Carboxyniederalkyl, z.B. Carboxymethyl oder 2-Carboxyäthyl, Niederalkoxycarbonylniederalkyl, z.B. Methoxycarbonylmethyl oder Aethoxycarbonylmethyl, Sulfoniederälkyl, z.B. Sulfomethyl oder 2-Sulfoäthyl, Aminoniederalkyl, z.B. Aminomethyl oder 2-Aminoäthyl, Niederalkylaminoniederalkyl, z.B. Methylaminomethyl, Diniederalkylaminoniederalkyl, z.B. Dimethylaminomethyl oder 2-Dimethylaminoäthyl, Carbamoylniederalkyl, z.B. Carbamoylmethyl, Diniederalkylcarbamoylniederalkyl, z.B. Dimethylcarbamoylmethyl, Sulfamoylniederalkyl, z.B. Sulfamoylmethyl, Diniederalkylsulfamoylniederalkyl, z.B. Dimethylsulfamoylmethyl, Hydroxyniederalkyl, z.B. Hydroxymethyl, oder durch Niederalkoxyniederalkyl, z.B. Methoxymethyl, substituiertes Pyrazolio.

Bevorzugte substituierte Pyrazoliogruppen R₁ sind beispielsweise 2-Niederalkyl-l-pyrazolio, z.B. 2-Methyl-, 2-Aethyl-, 2-Propyl- oder 2-tert.-Butyl-1-pyrazolio, 2-Niederalkenyl-l-pyrazolio, z.B. 2-Allyl- oder 2-Vinyl-l-pyrazolio, 2-Carboxyniederalkyl-1-pyrazolio, z.B. 2-Carboxymethyl-1-pyrazolio, 1-Carboxyniederalkyl-1-pyrazolio, z.B. 1-Carboxymethyl-l-pyrazolio, 2-Niederalkoxycarbonylniederalkyl-1-pyrazolio, z.B. 2-Methoxycarbonylmethyl-1- oder 2-Aethoxycarbonylmethyl-l-pyrazolio, 2-Sulfoniederalkyl-l-pyrazolio, z.B. 2-Sulfomethyl-l-pyrazolio, 2-Aminoniederalkyl-l-pyrazolio, z.B. 2-(2-Aminoäthyl)-l-pyrazolio, 2-Diniederalkylaminonie- deralkyl-1-pyrazolio, z.B. 2-(2-Dimethylaminoäthyl)-l-pyrazolio, 2-Carbamoylniederalkyl-1-pyrazolio, z.B. 2-Carbamoylmethyl-l-pyrazolio, 2-Diniederalkylcarbamoylniederalkyl-l-pyrazolio, z.B. 2-Dimethylcarbamoylmethyl-l-pyrazolio, 2-Sulfamoylniederalkyl-1-pyrazolio, z.B. 2-Sulfamoylmethyl-l-pyrazolio, 2-Hydroxyniederalkyl-1-pyrazolio, z.B. 2-Hydroxymethyl-l-pyrazolio, und 2-Niederalkoxyniederalkyl-1-pyrazolio, z.B. 2-Methoxymethyl-1-pyrazolio.

Eine unsubstituierte oder substituierte Triazoliogruppe R₁ ist beispielsweise Triazolio oder durch Niederalkyl, z.B. Methyl, Aethyl, Propyl oder tert.-Butyl, Niederalkenyl, z.B. Vinyl oder Allyl, Carboxyniederalkyl, z.B. Carboxymethyl oder 2-Carboxyäthyl, Sulfoniederalkyl, z.B. Sulfomethyl oder 2-Sulfoäthyl, Aminoniederalkyl, z.B. Aminomethyl oder 2-Aminoäthyl, Diniederalkylaminoniederalkyl, z.B. Dimethylaminomethyl oder 2-Dimethylaminoäthyl, Carbamoylniederalkyl, z.B. Carbamoylmethyl, Sulfamoylniederalkyl, z.B. Sulfamoylmethyl, Hydroxyniederalkyl, z.B. Hydroxymethyl, oder durch Niederalkoxyniederalkyl, z.B. Methoxymethyl, substituiertes Triazolio.

Bevorzugte substituierte Triazoliogruppen R₁ sind beispielsweise 3-Niederalkyl-1-triazolio, z.B. 3-Methyl-, 3-Aethyl-, 3-Propyl-, oder 3-tert.-Butyl-l-triazolio, 3-Niederalkenyl-l-triazolio, z.B. 3-Vinyl- oder 3-Allyl-l-triazolio, 3-Carboxyniederalkyl-l-triazolio, z.B. 3-Carboxymethyl-l-triazolio, 3-Sulfoniederalkyl-1-triazolio, z.B. 3-Sulfomethyl-l-triazolio, 3-Aminoniederalkyl-l-triazolio, z.B. 3-(2-Aminoäthyl)-l-triazolio, 3-Diniederalkylaminoniederalkyl-l-triazolio, z.B. 3-(2-Dimethylaminoäthyl)-1-triazolio, 3-Carbamoylniederalkyl-l-triazolio, z.B. 3-Carbamoylmethyl-l-triazolio, 3-Sulfamoylniederalkyl-l-triazolio, z.B. 3-Sulfamoylmethyl-1-triazolio, 3-Hydroxyniederalkyl-1-triazolio, z.B. 3-Hydroxymethyl-1-triazolio, und 3-Niederalkoxyniederalkyl-l-triazolio, z.B. 3-Methoxymethyl-1-triazolio.

Eine Triniederalkylammoniogruppe R₁ ist beispielsweise Trimethylammonio, Triäthylammonio oder Dimethyläthylammonio.

In einer Pyridiniogruppe der Formel A befindet sich R^{a} in 2-, 3-oder 4-Stellung des Pyridinrings.

In einer Pyridiniogruppe der Formel A bedeutet durch Cycloalkyl, Phenyl, Hydroxy, Niederalkoxy, Halogen, Cyano, Carbamoyl, Carboxyl oder Sulfo substituiertes Niederalkyl R^{a} beispielsweise Cyclopropyl-, Cyclopentyl-, oder Cyclohexylmethyl, Cyclopropyl-, Cyclopentyl- oder Cyclohexyl-1,1- oder -1,2-äthyl, Benzyl, 1- oder 2-Phenyläthyl, Diphenylmethyl oder Trityl, Hydroxymethyl, 1- oder 2-Hydroxyäthyl, Methoxymethyl, Aethoxymethyl oder Methoxy-1,1- oder 1,2-äthyl, Chlormethyl, Trifluormethyl, 2-Chlor-, 2-Brom- oder 2-Jodäthyl, 2,2,2-Trifluor- oder 2,2,2-Trichloräthyl, Cyanomethyl, 1- oder 2-Cyanoäthyl, Carbamoylmethyl, 2-Carbamoyläthyl, Carboxymethyl oder 2-Carboxyäthyl, Sulfomethyl, 1-Sulfo- oder 2-Sulfoäthyl.

Unsubstituiertes oder durch Carboxyl substituiertes Niederalkenyl oder Niederalkylthio R^{a} ist beispielsweise Vinyl, Allyl, n-Propenyl, 1-, 2- oder 3-Butenyl,. 2-Carboxyvinyl oder 3-Carboxyallyl, Methylthio, Aethylthio, n-Propylthio, Isopropylthio oder n-Butylthio, 2-Carboxyvinylthio oder 3-Carboxyallylthio.

Unsubstituiertes oder durch Niederalkyl, Niederalkanoyl oder Aminobenzolsulfonyl monosubstituiertes Amino R^{a} ist beispielsweise Amino, Methylamino, Aethylamino oder n-Propylamino, Formylamino, Acetylamino oder Propionylamino oder 3- oder 4-Aminobenzolsulfonylamino.

Diniederalkylamino R^{a} ist beispielsweise Dimethylamino, Diäthylamino oder Di-n-Propylamino.

Durch Niederalkyl, Hydroxyniederalkyl, Niederalkoxy, Hydroxy oder Cyano monosubstituiertes Carbamoyl R ist beispielsweise Methylcarbamoyl oder Aethylcarbamoyl, Hydroxymethylcarbamoyl oder 1- oder 2-Hydroxyäthylcarbamoyl, Methoxycarbamoyl oder Aethoxycarbamoyl, N-Hydroxycarbamoyl oder N-Cyanocarbamoyl.

Diniederalkylcarbamoyl R^{a} ist beispielsweise Dimethylcarbamoyl oder Diäthylcarbamoyl.

Cycloalkyl R^{a} ist beispielsweise Cyclopropyl, Cyclobutyl, Cyclopentyl oder Cyclohexyl.

Niederalkoxy R^{a} ist beispielsweise Methoxy, Aethoxy oder Propoxy.

Halogen R^{a} ist beispielsweise Fluor, Chlor oder Brom. Niederalkoxycarbonyl R^{a} ist beispielsweise Methoxycarbonyl oder Aethoxycarbonyl.

Niederalkanoyloxy R^{a} ist z.B. Formyloxy oder Acetoxy. Niederalkanoyl R^{a} ist z.B. Formyl, Acetyl, Propionyl, Isobutyryl oder n-Butyryl.

Hydroxysulfoniederalkyl R ist beispielsweise Hydroxysulfomethyl, 1-Hydroxy-2-sulfoäthyl, 2-Hydroxy-1-sulfoäthyl oder 2-Hydroxy-2-sulfoäthyl.

Pyridiniogruppen der Formel A, worin R die vorstehend genannten Bedeutungen hat, R^{b} die Bedeutungen von R^{a} hat, Carbamoyl oder Wasserstoff bedeutet und R^{c} Wasserstoff bedeutet, sind bevorzugt.

Bevorzugte Pyridiniogruppen der Formel A sind beispielsweise: Hydroxyniederalkylpyridinio, z.B. 3- oder 4-Hydroxymethylpyridinio, Niederalkoxyniederalkylpyridinio, z.B. 4-Methoxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanohalogenmethylpyridinio, z.B. 3-Cyano-4-trifluormethylpyridinio und Aminocarboxypyridinio, z.B. 2-Amino-3-carboxypyridinio.

Die Gruppe der Formel =N-O-R₂ liegt in der syn(oder Z)-Form oder in der anti(oder E)-Form vor, wobei die syn(oder Z)-Form bevorzugt ist.

Eine Niederalkylgruppe R₂ enthält vorzugsweise 1-4 C-Atome und ist beispielsweise Aethyl, Propyl, Butyl oder insbesondere Methyl.

Eine Cycloalkylgruppe R₂ enthält vorzugsweise 3-8, in erster Linie 3-6, Ringglieder und ist z.B. Cyclobutyl, Cyclopentyl oder Cyclohexyl und insbesondere Cyclopropyl.

Substituenten von substituiertem Niederalkyl oder Cycloalkyl R₂ sind u.a. gegebenenfalls veräthertes Hydroxy, z.B. Niederalkoxy, primäres, sekundäres oder tertiäres Amino Am, z.B. Amino oder Diniederalkylamino, gegebenenfalls funktionell abgewandeltes, z.B. verestertes, amidiertes oder geschütztes, Carboxyl oder Sulfo, sowie gegebenenfalls durch Niederalkyl N-substituiertes Ureidocarbonyl. Bevorzugt sind die substituierten Niederalkyl- und Cycloalkylgruppen durch eine Carboxyl- oder Sulfogruppe substituiert, wobei diese bevorzugt am Kohlenstoffatom, das mit dem Sauerstoffatom der Oxyiminogruppe verbunden ist, stehen.

Solche substituierte Niederalkyl- oder Cycloalkylgruppen R₂ sind beispielsweise 2-Aminoäthyl, 2-Dimethylaminoäthyl, Carboxymethyl, 1- oder 2-Carboxyäthyl, 1-,. 2- oder 3-Carboxyprop-1-yl, 1- oder 2-Carboxyprop-2-yl, 1-Carboxybut-1-yl, 1-Carboxycycloprop-1-yl und 1-Carboxycyclobut-1-yl, sowie entsprechende durch Sulfo substituierte Niederalkyl- und Cycloalkylgruppen.

Die Carboxy- und Sulfogruppen im Rest R₂ können beispielsweise durch Niederalkyl, wie Methyl oder Aethyl, oder durch eine physiologisch abspaltbare Gruppe, z.B. durch Pivaloyloxymethyl, verestert oder durch NH₃,ein primäres oder sekundäres Amin, z.B. ein Mono- oder Diniederalkylamin, z.B. Methyl- oder Aethylamin oder Dimethyl- oder Diäthylamia,amidiert sein oder durch die weiter unten genannten Schutzgruppen geschützt sein.

Gegebenenfalls substituiertes Carbamoyl als Rest Rist beispielsweise eine Gruppe -C(=O)-NHR, worin R Wasserstoff, Niederalkyl, z.B. Methyl, Aethyl oder l- oder 2-Propyl, gegebenenfalls geschütztes Carboxyniederalkyl, z.B. Carboxymethyl, 1- oder 2-Carboxyäthyl oder 1-, 2- oder 3-Carboxypropyl, worin Carboxy durch eine der üblichen Carboxylschutzgruppen geschützt oder beispielsweise durch Niederalkyl, z.B. Methyl, Aechyl, n- oder iso-Propyl, oder n- oder tert.-Butyl verestert sein kann, gegebenenfalls geschütztes Sulfoniederalkyl, z.B. Sulfomethyl,l- oder 2-Sulfoäthyl oder 1-, 2- oder 3-Sulfopropyl, worin Sulfo durch eine der üblichen Sulfoschutzgruppen geschützt oder beispielsweise durch Niederalkyl, z.B. Methyl oder Aethyl, verestert sein kann, gegebenenfalls geschütztes Hydroxyniederalkyl, z.B. Hydroxymethyl, 2-Hydroxyäthyl oder 2- oder 3-Hydroxypropyl, worin Hydroxy durch eine der üblichen Hydroxysehutzgruppen geschützt oder beispielsweise acyliert, z.B. acetyliert, sein kann, gegebenenfalls geschütztes Aminoniederalkyl, z.B. 2-Aminoäthyl, 2-oder 3-Aminopropyl oder 2-, 3- oder 4-Aminobutyl, worin Amino durch eine der üblichen Aminoschutzgruppen geschützt oder beispielsweise acyliert, z.B. acetyliert, sein kann, Arylniederalkyl, beispielsweise Phenylniederalkyl, z.B. Benzyl oder 1- oder 2-Phenyläthyl, Halogenniederalkyl, beispielsweise Fluor-, Chlor- oder Bromniederalkyl, z.B. 2-Chloräthyl, 3-Chlorpropyl- oder 4-Chlorbutyl, Aryl, z.B. Phenyl, oder durch Niederalkyl, z.B. Methyl, Niederalkoxy, z.B. Methoxy, Halogen, z.B. Chlor oder Nitro, ein- bis dreifach substituiertes Phenyl bedeutet.

Wie oben erwähnt, können in Verbindungen der Formel I vorhandene funktionelle Gruppen, insbesondere die Carboxyl und Aminogruppen ferner die Hydroxy- oder Sulfogruppen, gegebenenfalls durch Schutzgruppen geschützt sein, wobei üblicherweise die in der Penicillin-, Cephalosporin- und Peptidchemie verwendeten Schutzgruppen zur Anwendung kommen.

Solche Schutzgruppen sind leicht, das heisst ohne dass unerwünschte Nebenreaktionen stattfinden, beispielsweise solvolytisch,- reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke,Academic Press, London, New York, 1965, sowie in "Methoden der organischen Chemie", Houben-Weyl, 4-Auflage, Bd.15/1, Georg Thieme Verlag, Stuttgart, 1974.

So sind Carboxylgruppen z.B. in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.Butyloxycarbonyl, Arylmethoxycarbonyl, mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Nitrobenzyloxycarbonyl oder 4-Methoxybenzyloxycarbonyl, oder, z.B.. wie oben erwähnt, gegebenenfalls substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxyearbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B.Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphatischen oder aromatischen Kohlenwasserstoffrest mit z.B. bis zu 15 Kohlenstoffatomen bedeuten, beispielsweise gegebenenfalls substituiertes Niederalkyl, Phenylniederalkyl, Cycloalkyl oder Phenyl, z.B.. 2-Triniederalkylsilyläthoxycarbonyl, wie 2-Trimethylsilyläthoxycarbonyl oder 2-(Di-n-butylmethylsilyl)-äthoxycarbonyl, oder 2-Triarylsilyläthoxycarbonyl, wie 2-Triphenylsilyläthoxycarbonyl.

Weitere, in veresterter Form vorliegende, geschützte Carboxylgruppen sind Silyloxycarbonyl-, insbesondere organische Silyloxycarbonylgruppen, ferner Stannyloxycarbonylgruppen. In diesen Gruppen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.-butylsilyl, Niederalkoxyniederalkylhalogensilyl, z.B. Methoxymethylchlorsilyl, oder Diniederalkylhalogensilyl, z.B. Dimethylchlorsilyl, oder entsprechend substituierte Stannylverbindungen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitrobenzyloxycarbonyl oder Diphenylmethoxycarbonyl.

Eine geschützte Aminogruppe, wie eine entsprechende Gruppe Am, kann z.B. in Form einer leicht spaltbaren Acylamino-, Arylmethylamino-, verätherten Mercaptoamino-, 2-Acylniederalk-1-en-1-ylamino-, Silyl- oder Stannylaminogruppe oder als Azidogruppe vorliegen.

In einer leicht spaltbaren Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder einer gegebenenfalls,z.B. durch Halogen, Niederalkoxy oder Nitro, substituierten Benzoesäure, oder eines Kohlensäurehalbesters. Solche Acylgruppen sind . beispielsweise Niederalkanoyl, wie Formyl, Acetyl, oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro,substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niedecalkoxycarbonyl, insbesondere tert.-Niederalkoxycarbonyl, z.B. tert.-Butyloxycarbonyl, Arylmethoxycarbonyl mit einem oder zwei Arylresten, die gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder gegebenenfalls substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls,-z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen bedeuten, beispielsweise gegebenenfalls substituiertes Niederalkyl, Phenylniederalkyl, Cycloalkyl oder Phenyl, z.B.. 2-Triniederalkylsilyläthoxycarbonyl, wie 2-Trimethylsilyläthoxycarbonyl oder 2-(Di-n-butylmethylsilyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Diphenylniederalkylphosphoryl, z.B. Dibenzylphosphoryl oder Di-4-nitrobenzylphosphoryl, gegebenenfalls substituiertes Phenyloxyphenylphosphonyl, z.B. Phenyloxyphenylphosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro,substituiertes Phenyl ist. Eine entsprechende Aminoschutzgruppe ist z.B. 4-Nitrophenylthio.

In einem als Aminoschutzgruppe verwendbaren 2-Acylniederalk-1-en-1-ylrest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro,substituierten Benzoesäure oder insbesondere eines Kohlensäurehalbesters, wie eines Kohlensäure-niederalkylhalbesters. Entsprechende Schutzgruppen sind in erster Linie 1-Niederalkanoyl- prop-l-en-2-yl, z.B. 1-Acetylprop-1-en-2-yl, oder 1-Niederalkoxycar- bonylprop-l-en-2-yl, z.B. l-Aethoxycarbonylprop-l-en-2-yl.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl- tert.-butylsilyl, Niederalkoxyniederalkylhalogensilyl, z.B. Methoxymethylchlorsilyl, oder Diniederalkylhalogensilyl, z.B. Dimethylchlorsilyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfons äiren, wie p-Töluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitrobenzyloxycarbonyl oder Diphenylmethoxycarbonyl, oder .2-Halogenniederalkoxycarbonyl, wie 2,2,2-Trichloräthoxycarbonyl, ferner Trityl oder Formyl.

Hydroxyschutzgruppen sind z.B. Acylreste, wie gegebenenfalls, z.B. durch Halogen, substituiertes Niederalkanoyl, wie 2,2-Dichloracetyl, oder insbesondere die im Zusammenhang mit einer geschützten Aminogruppe genannten Acylreste von Kohlensäurehalbestern, insbesondere 2,2,2-Trichloräthoxycarbonyl, oder organische Silyl- oder Stannylreste , ferner leicht abspaltbare veräthernde Gruppen, wie tert.-Niederalkyl, z.B. tert.-Butyl, 2-oxa- oder 2-thiaaliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxyäthyl, 1-Aethoxyäthyl, 1-Methylthiomethyl, i-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste, z.B. Halogen, wie Chlor, Niederalkoxy, wie Methoxy und/oder Nitro , in Frage kommen.

Eine geschützte Sulfogruppe ist in erster Linie eine veresterte, wie mit einem aliphatischen, cycloaliphatischen, cycloaliphatischaliphatischen, aromatischen oder araliphatischen Alkohol, z.B. einem Niederalkanol, oder mit einem Silyl- oder Stannylrest, wie Triniederalkylsilyl, veresterte Sulfogruppe. In einer Sulfogruppe kann die Hydroxygruppe,beispielsweise wie die Hydroxygruppe in einer veresterten Carboxygruppe, veräthert sein.

Salze von erfindungsgemässen Verbindungen sind in erster Linie pharmazeutisch verwendbare, nicht-toxische Salze, wie diejenigen von Verbindungen der Formel I mit sauren Gruppen, z.B. mit einer freien Carboxyl- und Sulfogruppe. Solche Salze sind in erster Linie Metall-oder Ammoniumsalze, wie Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatischaliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethylpiperidin, Cycloalkylamine, z.B. Dicyclohexylamin, oder Benzylamine, z.B. N,N-Dibenzyläthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren Gruppe, z.B. einer freien Carboxylgruppe, und einer basischen Gruppe, z.B. einer Aminogruppe, können auch in Form von inneren Salzen, d.h. in zwitterionischer Form, vorliegen, oder es kann ein Teil des Moleküls als inneres Salz und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel I, worin die funktionellen Gruppen in freier, d.h. nicht in geschützter Form, vorliegen, und ihre pharmazeutisch verwendbaren, nicht-toxischen Salze sind wertvolle, antibiotisch wirksame Substanzen, die insbesondere als antibakterielle Antibiotika verwendet werden können. Beispielsweise sind sie in vitro gegen grampositive Keime, beispielsweise gegen grampositive Kokken, z.B. Staphylococcus aureus, Streptokokken, z.B. Streptococcus pyogenes, Streptococcus pneumoniae und Streptococcus faecalis, gegen grampositive anaerobe Keime, z.B. Clostridium perfringens und Peptostreptococcus anaerobius in Konzentrationen von ca. 0,05 µg/ml bis ca. 64 µg/ml, gegen gramnegative Keime, beispielsweise Enterobacteriaceae, z.B. Escherichia coli, Proteus spp.., Klebsiella pneumoniae, Serratia marcescens und Enterobacter cloacae, sowie Pseudomonas aeruginosa, Haemophilus influenzae und Neisseria spp. und gegen anaerobe gramnegative Keime, z.B. Bacteroides fragilis, in Konzentrationen von ca. 0,005 bis ca. 32 µg/ml, ferner auch gegen andere grampositive und gramnegative Keime wirksam. In vivo, bei subkutaner Anwendung an der Maus, sind sie gegen systemische Infektionen, welche durch grampositive Keime wie Staphylococcus aureus verursacht werden, in einem Dosisbereich von ca. 5 bis ca. 100 mg/kg und gegen systemische Infektionen, welche durch gramnegative Keime wie Enterobacteriaceae, oder Pseudomonas aeruginosa verursacht werden, in einem Dosisbereich von ca. 0,9 mg/kg bis ca. 100 mg/kg wirksam.

### Versuchsbericht

I. Für die folgenden Verbindungen wurde die antibiotische Wirksamkeit getestet:
1. 7ß-[2-(5-Amino-1,2,4-thiadiazol-3-yl)-2-Z-methoxyiminoacetamido]-3-(4-carboxypyridiniomethyl)-3-cephem-4-carboxylat (Beispiel 1);
2. 7β-[2-(5-Amino-1,2,4-thiadiazol-3-yl)-2-Z-2-carboxyprop-2-yloxy- iminoacetamido]-3-(3-carboxymethylpyridiniomethyl)-3-cephem-4-carboxylat (Beispiel 2);
3. 7ß-[2-(5-Amino-1,2,4-thiadiazol-3-yl)-2-Z-2-carboxyprop-2-yloxy- iminoacetamido]-3-(2-methyl-1-pyrazoliomethyl)-3-cephem-4-carboxylat (Beispiel 6);
4. 7β-[2-(5-Amino-1,2,4-thiadiazol-3-yl)-2-Z-methoxyiminoacetamido]-3-(2-methyl-1-pyrazoliomethyl)-3-cephem-4-carboxylat (Beispiel 8);
5. 7β-[2-(5-Amino-1,2,4-thiadiazol-3-yl)-2-Z-methoxyiminoacetamido]-3--(3-brompyridiniomethyl)-3-cephem-4-carboxylat (Beispiel 9);
6. 7ß-[2-(5-Amino-1,2,4-thiadiazol-3-yl)-2-Z-methoxyiminoacetamido]-3-(4-hydroxymethylpyridiniomethyl)-3-cephem-4-carboxylat (Beispiel 11);

### II. Methodik

A. Die antibiotische Aktivität der Testverbindungen in vitro wurde durch die Agarverdünnungsmethode nach Ericson H.M. und Sherris S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen MIC (=minimum inhibiting concentration) werden in Mikrogramm pro Milliliter µg/ml) für die geprüften Verbindungen in der Tabelle 1 angegeben.

B. Die chemotherapeutische Wirksamkeit in vivo gegen systemische Infektionen in weiblichen SPF, MF₂ Mäusen wurde nach der Methode von Zak, 0., et al., 1979, Drugs Exptl. Clin. Res. 5, 45-59, ermittelt. Die gefundenen ED₅₀-Werte in Milligramm Substanz pro Kilogramm Maus (mg/kg) gegen eine Anzahl von Mikroorganismen werden für die subcutan (s.c.) applizierten Testverbindungen in der Tabelle 2 angegeben.

### III. Versuchsergebnisse

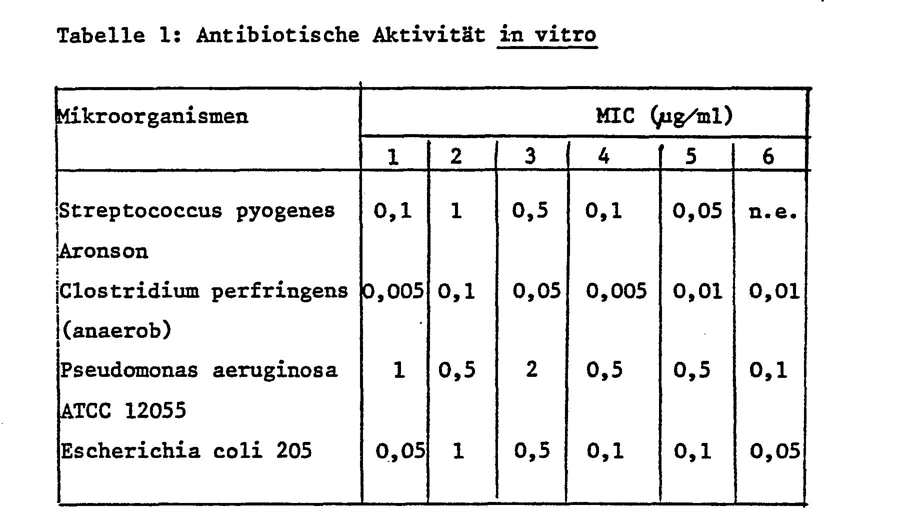

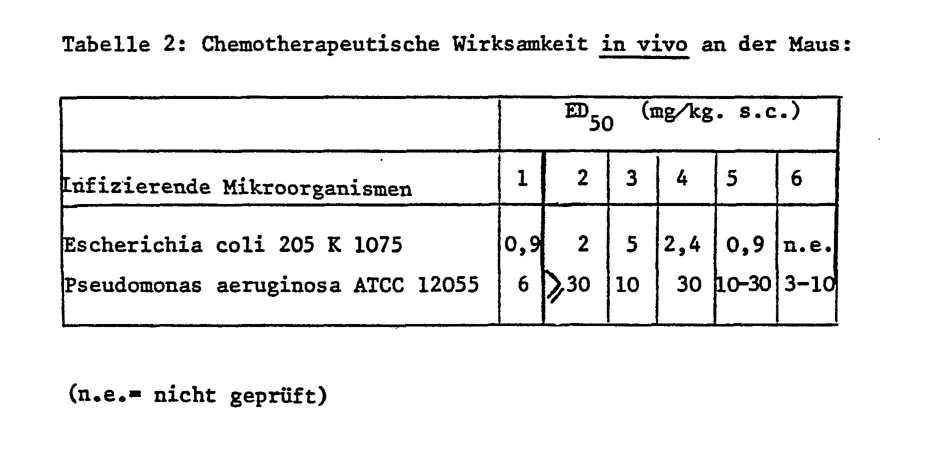

Die neuen Verbindungen der Formel I können deshalb, z.B. in Form von antibiotisch wirksamen Präparaten, zur Behandlung von Infektionen, welche durch grampositive oder gramnegative Bakterien und Kokken, insbesondere durch Enterobakterien, wie Serratia marcescens oder Escherichia coli, sowie durch Pseudomonas verursacht werden, Verwendung finden.

Verbindungen der Formel I, worin die funktionellen Gruppen geschützt sind, werden als Zwischenprodukte zur Herstellung von Verbindungen der Formel I verwendet, worin die funktionellen Gruppen in freier Form vorliegen.

Die Erfindung betrifft insbesondere Verbindungen der Formel I, worin n für 0 steht, Am Amino, Niederalkylamino, Diniederalkylamino oder geschütztes Amino, T einen Thiadiazolrest, welcher an einem Kohlenstoffatom mit Am und am anderen Kohlenstoffatom mit der Gruppe der Formel -C(=N-O-R₂)- verbunden ist, R1 2-Niederalkyl-l-pyrazolio, 2-Carboxyniederalkyl-l-pyrazolio, 1-Carboxyniederalkyl-l-pyrazolio, 2-Niederalkoxycarbonylniederalkyl-l-pyrazolio, 3-Niederalkyl-1-triazolio oder eine Pyridiniogruppe der Formel A, worin R^{a} durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro oder Hydroxysulfoniederalkyl bedeutet, R Carbamoyl oder Wasserstoff bedeutet oder die Bedeutungen von R^{a} hat und R^{c} Wasserstoff bedeutet,und R₂ Wasserstoff, unsubstituiertes oder substituiertes Niederalkyl oder Cycloalkyl oder unsubstituiertes oder substituiertes Carbamoyl bedeutet, wobei die Gruppe der Formel =N-O-R₂ insbesondere die syn(oder Z)-Form aufweist, sowie Salze, insbesondere pharmazeutisch verwendbare Salze von solchen Verbindungen der Formel I, die salzbildende Gruppen aufweisen.

Die Erfindung betrifft in erster Linie Verbindungen der Formel I, worin n für 0 steht, Am Amino, Niederalkylamino, z.B. Methylamino, oder geschütztes Amino, T einen Thiadiazolrest, welcher an einem Kohlenstoffatom mit Am, und am anderen Kohlenstoffatom mit der Gruppe der Formel -C(=N-O-R₂)- verbunden ist, R₁ 2-Niederalkyl-l-pyrazolio, z.B. 2-Methyl-l-pyrazolio, 2-Carboxyniederalkyl-l-pyrazolio, z.B. 2-Carboxymethyl-l-pyrazolio, 1-Carboxyniederalkyl-l-pyrazolio, z.B. 1-Carboxymethyl-l-pyrazolio, 2-Niederalkoxycarbonylniederalkyl-l-pyrazolio, z.B. 2-Methoxycarbonylmethyl-l-pyrazolio, 3-Niederalkyl-l-triazolio, z.B. 3-Methyl-l-triazolio oder eine Pyridiniogruppe der Formel A, beispielsweise Hydroxyniederalkylpyridinio, z.B. 3- oder 4-Hydroxymethylpyridinio, Niederalkoxyniederalkylpyridinio, z.B. 4-Methoxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanohalogenmethylpyridinio, z.B. 3-Cyano-4-trifluormethylpyridinio oder Aminocarboxypyridinio, z.B. 2-Amino-3-carboxypyridinio und R₂ Wasserstoff, Niederalkyl, z.B. Methyl, Carboxyniederalkyl, z.B. 2-Carboxy-2-propyl, Carbamoyl, Niederalkylcarbamoyl, z.B. Methylcarbamoyl, oder Aminoniederalkyl, z.B.. 2-Aminoäthyl, bedeuten, wobei die Gruppe der Formel =N-O-R₂ die syn(oder Z)-Form aufweist, sowie Salze, insbesondere pharmazeutisch verwendbare Salze von solchen Verbindungen der Formel I, die salzbildende Gruppen aufweisen.

Besonders hervorzuheben sind Verbindungen der Formel I, worin n für 0 steht, Am Amino, T einen 1.,2,4-Thiadiazolrest, welcher in 5-Stellung mit Am und in 3-Stellung mit der Gruppe der Formel -C(=N-O-R₂)- verbunden ist, R₁ 2-Niederalkyl-l-pyrazolio, z.B. 2-Methyl-l-pyrazolio, 2-Carboxyniederalkyl-l-pyrazolio, z.B. 2-Carboxymethyl-l-pyrazolio, 2-Niederalkoxycarbonylniederalkyl-1-pyrazolio, z.B. 2-Methoxycarbonylmethyl-l-pyrazolio, 3-Niederalkyl-l-triazolio, z.B. 3-Methyl-l-triazolio, oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Sulfoniederalkylpyridinio, z.B. 4-(2-Sulfoäthylpyridinio), Halogenpyridinio, z.B. 3-Brompyridinio, Carboxypyridinio, z.B. 4-Carboxypyridinio, Sulfopyridinio, z.B. 3-Sulfopyridinio oder Aminocarbamoylpyridinio, z.B. 2-Amino-5-carbamoylpyridinio, und R₂ Wasserstoff, Niederalkyl, z.B. Methyl, Carboxyniederalkyl, z.B. 2-Caboxy-2-propyl, Carbamoyl oder Niederalkylcarbamoyl, z.B. Methylcarbamoyl, bedeuten, wobei die Gruppe der Formel =N-O-R₂ die syn(oder Z)-Form aufweist, sowie Salze, in erster Linie pharmazeutisch verwendbare Salze von Verbindungen der Formel I.

Ganz besonders hervorzuheben sind Verbindungen der Formel I, worin n für 0 steht, Am Amino, T einen 1,2,4-Thiadiazolrest, welcher in 5-Stellung mit Am und in 3-Stellung mit der Gruppe der Formel -C(-N-O-R₂)- verbunden ist, R₁ 2-Niederalkyl-I-pyrazolio, z.B. 2-Methyl-l-pyrazolio oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Halogenpyridinio, z.B. 3-Brompyridinio, oder Carboxypyridinio, z.B. 4-Carboxypyridinio, und R₂ Niederalkyl, z.B. Methyl oder Carboxyniederalkyl, z.B. 2-Carboxy-2-propyl oder Aminocarbamoylpyridinio, z.B. 2-Amino-5-carbamoylpyridinio bedeuten, wobei die Gruppe der Formel =N-O-R₂ die syn(oder Z)-Form aufweist, sowie Salze, in erster Linie pharmazeutisch verwendbare Salze von Verbindungen der Formel I.

Vor allem betrifft die vorliegende Erfindung die in den Beispielen beschriebenen Verbindungen der Formel I, sowie deren Salze, insbesondere deren pharmazeutisch verwendbaren Salze.

Vor allem sind folgende Verbindungen der Formel I hervorzuheben:
7β-[2-(5-Amino-1,2,4-thiadiazol-3-yl)-2-Z-methoxyiminoacetamido]-3-(4-carboxypyridiniomethyl)-3-cephem-4-carboxylat,
7β-[2-(5-Amino-1,2,4-thiadiazol-3-yl)-2-Z-2-carboxyprop-2-yloxy- iminoacetamido]-3-(3-carboxymethylpyridiniomethyl)-3-cephem-4-carboxylat,
7ß-[2-(5-Amino-I,2,4-thiadiazol-3-yl)-2-Z-2-carboxyprop-2-yloxy- iminoacetamido]-3-(2-methyl-l-pyrazoliomethyl)-3-cephem-4-carboxylat,
7β-[2-(5-Amino-1,2,4-thiadiazol-3-yl)-2-Z-methoxyiminoacetamido]-3-(2-methyl-l-pyrazoliomethyl)-3-cephem-4-carboxylat,
7β-[2-(5-Amino-1,2,4-thiadiazol-3-yl)-2-Z-methoxyiminoacetamido]-3-(3-brompyridiniomethyl)-3-cephem-4-carboxylat, und
7ß-[2-(5-Amino-1,2,4-thiadiazol-3-yl)-2-Z-methoxyiminoacetamido]-3-(4-hydroxymethylpyridiniomethyl)-3-cephem-4-carboxylat.

Verbindungen der Formel I und Salze von solchen Verbindungen, die eine salzbildende Gruppe aufweisen, werden beispielsweise hergestellt, indem man
a) in einer Verbindung der Formel 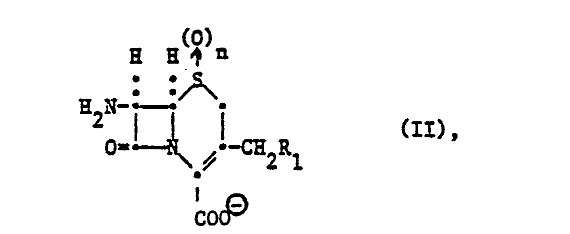 worin der Index n für 0 oder 1 steht, R₁ die unter Formel I genannten Bedeutungen hat, die 7ß-Aminogruppe gegebenenfalls durch eine die Acylierungsreaktion erlaubende Gruppe geschützt ist und die 4-Carboxygruppe gegebenenfalls geschützt ist, die 7ß-Aminogruppe durch Umsetzung mit einem den Acylrest einer Carbonsäure der Formel 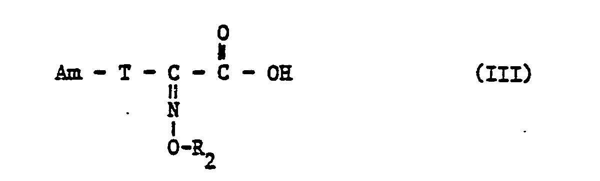 einführenden Acylierungsmittel, worin Am, T und R die unter Formel I genannten Bedeutungen haben und vorhandene funktionelle Gruppen gegebenenfalls geschützt sind, acyliert oder
b) zur Herstellung einer Verbindung der Formel I, worin n für 0 steht, eine 2-Cephemverbindung der Formel 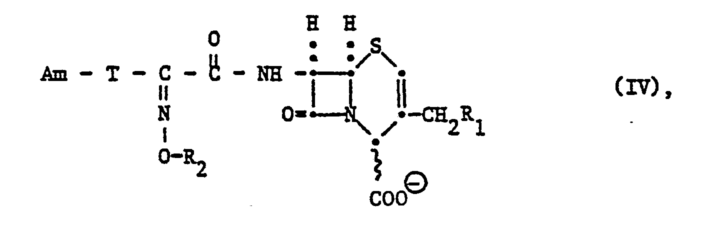 worin Am, T, R₁ und R₂ die unter Formel I genannten Bedeutungen haben und worin die 4-Carboxylgruppe sowie weitere funktionelle Gruppen gegebenenfalls geschützt sind, zur entsprechenden 3-Cephemverbindung isomerisiert. oder
c) eine Verbindung der Formel 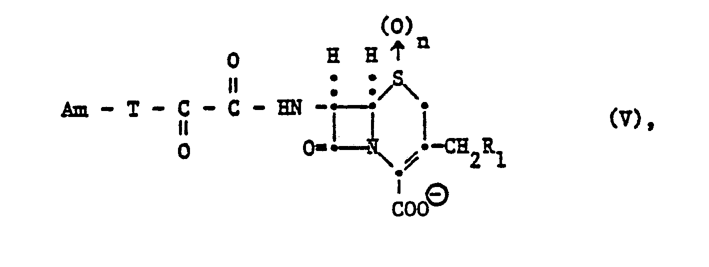 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Hydroxylaminderivat der Formel H₂N-O-R₂ (VI), worin R₂ die unter Formel I genannten Bedeutungen hat und funktionelle Gruppen gegebenenfalls geschützt sind, umsetzt oder
d) zur Herstellung einer Verbindung der Formel I, worin R₂ Wasserstoff bedeutet, eine Verbindung der Formel 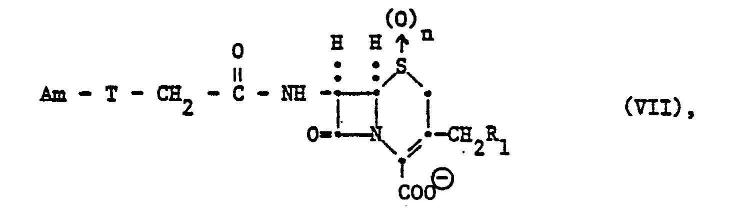 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Nitrosierungsmittel behandelt oder
e) zur Herstellung einer Verbindung der Formel I, worin Am eine freie oder eine sekundäre Aminogruppe, T einen 1,2,4-Thiadiazolrest darstellt, der in 5-Stellung mit Am und in 3-Stellung mit der Gruppe der Formel -C(=N-O-R₂)- verbunden ist, eine Verbindung der Formel 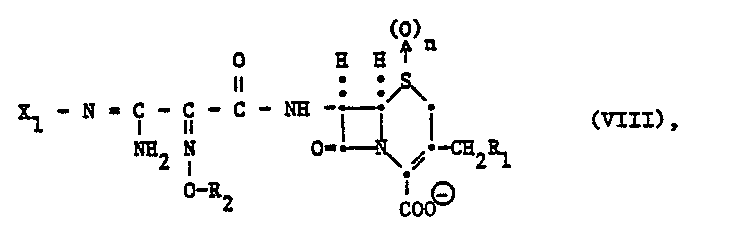 worin n für 0 oder 1 steht, X₁ Wasserstoff, Halogen oder Hydroxy bedeutet und R₁ die unter Formel I genannten Bedeutungen hat und worin die 4-Carboxylgruppe, sowie weitere funktionelle Gruppen gegebenenfalls geschützt sind, mit einem Salz der Rhodanwasserstoffsäure, bzw. mit einem Isorhodanwasserstoffsäureester oder, falls Xₗ Wasserstoff bedeutet, mit Dirhodan behandelt oder
f) eine Verbindung der Formel 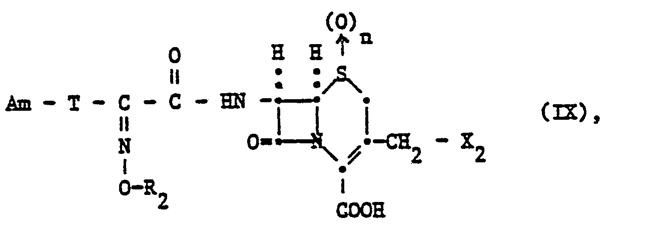 worin n für 0 oder 1 steht, Am, T und R₂ die unter Formel I genannten Bedeutungen haben und X₂ einen durch nucleophile Substitution ersetzbaren Rest darstellt und worin die 4-Carboxylgruppe sowie weitere funktionelle Gruppen gegebenenfalls geschützt sind, mit einer dem Rest R₁ entsprechenden organischen tertiären Base umsetzt oder
g) zur Herstellung von Verbindungen der Formel I, worin Am eine primäre, sekundäre oder tertiäre Aminogruppe ist, eine Verbindung der Formel 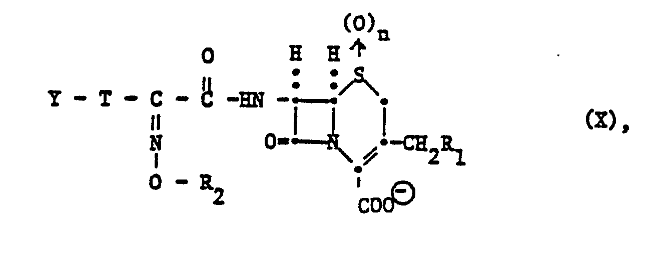 worin n für 0 oder 1 steht, T, R₁ und R₂ die unter Formel I genannten Bedeutungen haben und Y Halogen bedeutet, mit Ammoniak oder einem primären oder sekundären Amin Am-H oder einem Metallamid davon umsetzt und, wenn erwünscht, eine erhältliche Verbindung der Formel I in eine andere Verbindung der Formel I umwandelt und/oder, wenn erwünscht, eine erhältliche Verbindung der Formel I, worin n 0 bedeutet, in eine Verbindung der Formel I überführt, worin n 1 bedeutet, und/oder eine Verbindung der Formel I, worin n 1 bedeutet, in eine Verbindung der Formel I überführt, worin n 0 bedeutet, und/oder in einer erhältlichen Verbindung der Formel I in geschützter Form vorliegende funktionelle Gruppen in die freien funktionellen Gruppen überführt und/oder ein erhältliches Salz in die freie Verbindung der Formel I oder in ein anderes Salz überführt, und/oder eine erhältliche freie Verbindung der Formel I mit einer salzbildenden Gruppe in ein Salz überführt und/oder ein erhältliches Gemisch von isomeren Verbindungen in die einzelnen Isomeren auftrennt.

### Verfahren a) (Acylierung):

In einem Ausgangsmaterial der Formel II ist die 7ß-Aminogruppe gegebenenfalls durch eine die Acylierung erlaubende Gruppe geschützt. Solche Gruppen sind beispielsweise organische Silyl- oder Stannylgruppen, ferner Ylidengruppen, die zusammen mit der Aminogruppe eine Schiff'sche Base bilden. Die genannten organischen Silyl- oder Stannylgruppen sind z.B. solche Gruppen, die auch mit einer Carboxylgruppe R₂ eine geschützte Carboxylgruppe zu bilden vermögen. Es sind dies in erster Linie Triniederalkylsilyl, insbesondere Trimethylsilyl. Bei der Silylierung oder Stannylierung zum Schutz einer 4-Carboxylgruppe in einem Ausgangsmaterial der Formel II kann bei Verwendung eines Ueberschusses des Silylierungs- oder Stannylierungsmittels, die 7ß-Aminogruppe ebenfalls silyliert oder stannyliert werden. Die genannten Ylidengruppen sind in erster Linie 1-Aryl-niederalkyliden-, insbesondere 1-Arylmethylengruppen, worin Aryl besonders für einen carbocyclischen, in erster Linie monocyclischen Arylrest steht, z.B. für gegebenenfalls durch Niederalkyl, Hydroxy, Niederalkoxy und/oder Nitro substituiertes Phenyl.

Weitere in einem Ausgangsmaterial der Formel II vorhandene funktionelle Gruppen können z.B. durch die obengenannten Schutzgruppen geschützt sein. Vorzugsweise sind die an der Acylierungsreaktion nicht teilnehmenden funktionellen Gruppen, insbesondere gegebenenfalls vorhandene acylierbare Amino-, Hydroxy- und Mercaptogruppen, entsprechend geschützt.

In einem Acylrest der Säure der Formel III kann eine vorhandene Aminogruppe auch in ionischer Form geschützt sein, d.h. das Ausgangsmaterial der Formel III mit einer solchen Aminogruppe kann in Form eines Säureadditionssalzes, vorzugsweise mit einer starken anorganischen Säure, wie einer Halogenwasserstoffsäure, z.B.. Salzsäure oder Schwefelsäure, verwendet werden.

Acylierungsmittel, welche den Acylrest einer Carbonsäure der Formel III einfuhren, sind beispielsweise die Carbonsäure selbst oder ihre reaktionsfähigen, funktionellen Derivate.

Falls eine freie Carbonsäure der Formel III, worin vorhandene funktionelle Gruppen ausser der an der Reaktion teilnehmenden 4-Carboxylgruppe geschützt sein können, zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylamino- propylcarbodiimid, geeigneten Carbonylverbindungen, beispielsweise Carbonyldiimidazol, oder 1,2-Oxazoliumverbindungen, wie 2-Aethyl-5-phenyl-l,2-oxazolium-3'-sulfonat oder 2-tert.-Butyl-5-methyl-1,2-oxazöliumperchlorat, oder einer geeigneten Acylaminoverbindung, z.B. 2-Aethoxy-1-äthoxycarbonyl-1,2-dihydrochinolin, durchgeführt.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, in einem Gemisch dieser Lösungsmittel mit Wasser oder in Wasser, wenn erwünscht oder notwendig, unter Kühlen oder Erwärmen, z.B. in einem Temperaturbereich von etwa -40°C bis etwa +100°C, bevorzugt von etwa -20°C bis etwa +50°C und/oder in einer Inertgas-, z.B. Stickstoffatmosphäre, durchgeführt.

Ein reaktionsfähiges funktionelles Derivat einer Carbonsäure der Formel III, worin vorhandene funktionelle Gruppen ausser der an der Reaktion beteiligten Carboxylgruppe geschützt sein können, ist in erster Linie ein Anhydrid einer solchen Säure, inklusive und vorzugsweise ein gemischtes Anhydrid. Gemischte Anhydride sind z.B. diejenigen mit anorganischen Säuren wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure, Diäthylphosphorsäure oder phosphoriger Säure, oder mit einer schwefelhaltigen Säure, z.B. Schwefelsäure oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere zur Reaktion mit der Aminogruppe geeignete Derivate einer Säure der Formel III, worin vorhandene funktionelle Gruppen ausser der an der Reaktion teilnehmenden Carboxylgruppe geschützt sein können, sind aktivierte Ester, beispielsweise Ester mit vinylogen Alkoholen, d.h. mit Enden, wie vinylogen Niederalkenolen oder ^{I}minomethylesterhalogeniden, z.B. Dimethyliminomethylesterchlorid, hergestellt aus der Carbonsäure der Formel III und z.B. Dimethyl-(1-chlor-äthyliden)- imiuiumchlorid der Formel (CH₃)₂N^{⊕}=C(Cl)CH₃ Cl^{⊖}, das man seinerseits z.B. aus N,N-Dimethylacetamid und Phosgen erhalten kann, Arylester, z.B. durch Halogen, wie Chlor, und/oder Nitro,substituierte Phenylester, z.B. 4-Nitrophenyl-, 2,3-Dinitrophenyl- oder Pentachlorphenylester, N-heteroaromatische Ester, wie N-Benztriazol-, z.B. 1-Benztriazolester, oder N-Diacyliminoester, wie N-Succinylimino- oder N-Phthalylimiuoester.

Die Acylierung mit einem reaktionsfähigen funktionellen Derivat einer Carbonsäure der Formel III, z.B. einem entsprechenden Anhydrid, insbesondere einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base,, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines 1,2-Niederalkylenoxids, wie Aethylenoxid oder Propylenoxid, durchgeführt.

Die obigen Acylierungen werden vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder einer Mischung davon, wenn notwendig oder erwünscht, bei erniedrigter oder erhöhter Temperatur, z.B. in einem Temperaturbereich von etwa -40°C bis etwa +100°C, bevorzugt von etwa -10°C bis etwa +50°C, und/oder in einer Inertgas-, z.B. Stickstoffatmosphäre.

Die Acylierung kann auch durch Verwendung eines geeigneten Derivates der Säure der Formel III in Gegenwart einer geeigneten Acylase erfolgen. Solche Acylasen sind bekannt und können durch eine Anzahl von Mikroorganismen gebildet werden, z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Ein in der Aeylierungsreaktion eingesetztes reaktionsfähiges funktionelles Derivat einer Säure der Formel III kann, wenn erwünscht, in situ gebildet werden. So kann man z.B. ein gemischtes Anhydrid herstellen, indem man eine Säure der Formel III, gegebenenfalls mit entsprechend geschützten funktionellen Gruppen, oder ein geeignetes Salz davon, z.B. ein Ammoniumsalz, welches z.B. mit einer organischen Base, wie Pyridin oder 4-Methylmorpholin, gebildet wird, oder ein Metall-, z.B. Alkalimetallsalz, mit einem geeigneten Säurederivat, beispielsweise einem entsprechenden Säurehalogenid, einer gegebenenfalls substituierten Niederalkancarbonsäure, z.B. Trichloracetylchlorid, einem Halbester eines Kohlensäurehalbhalogenids, z.B. Chlorameisensäureäthylester oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung in der Aeylierungsreaktion verwenden.

### Verfahren b) (Isomerisierung):

In einem 2-Cephem-Ausgangsmaterial der Formel IV weist die gegebenenfalls geschützte 4-Carboxylgruppe vorzugsweise die α-Konfiguration auf.

2-Cephem-Verbindungen der Formel IV können isomerisiert werden, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Ficoline, Collidine oder Lutidine, ferner Chinolin, tertiäre aromatische Basen, z.B. solche des Anilin -Typs, wie N,N-Diniederalkylaniline, z.B. N,N-Dimethylanilin oder N,N-Diäthylanilin, oder tertiäre aliphatische, azacycloaliphatische oder araliphatische Basen, wie Triniederalkylamine, z.B. Trimethylamin oder N,N-Diisopropyläthylamin, N-Niederalkylazacycloalkane, z.B. N-Methylpiperidin, oder N,N-Diniederalkyl- phenylniederalkylamine, z.B. N,N-Dimethylbenzylamin, sowie Gemische von solchen basischen Mitteln, wie das Gemisch einer Base vom Pyridin- typ, und eines Triniederalkylamins, z.B. Pyridin und Triäthylamin. Ferner können auch anorganische oder organische Salze von Basen, insbesondere von mittelstarken bis starken Basen mit schwachen Säuren, wie Alkalimetall- oder Ammoniumsalze von Niederalkancarbonsäuren, z.B. Natriumacetat, Triäthylammoniumacetat oder N-Methylpiperidinacetat, sowie andere analoge Basen oder Gemische von solchen basischen Mitteln verwendet werden.

Bei der Isomerisierung von 2-Cephem-Verbindungen der Formel IV mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei als Reaktionsmittel verwendete unter den Reaktionsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstoffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von 2-Cephem-verbindungen der Formel IV zur entsprechenden 3-Cephem-verbindung wird vorzugsweise durchgeführt, indem man diese in 1-Stellung oxidiert, wenn erwünscht, gegebenenfalls Isomerengemische der gebildeten 1-Oxide trennt, und die so erhältlichen 1-Oxide der entsprechenden 3-Cephemverbindungen reduziert.

Als geeignete Oxidationsmittel für die Oxidation in 1-Stelluag von 2-Cephem-verbindungen der Formel IV kommen anorganische Persäuren, die ein Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Dissoziationskonstante von wenigstens 10⁻⁵ in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens 10⁻⁵ durchgeführt werden, wobei man niedrige Konzentrationen, z.B. 1-2X und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalvsatoren durchgeführt werden. So kann z.B. die Oxidation mit Percarbonsäuren durch die Anwesenheit einer Säure mit einer Dissoziationskonstante von wenigstens 10⁻⁵ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20Z, wobei man auch grössere Ueberschüsse, d.h. bis zur 10-fachen Menge des Oxidationsmittels oder darüber verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-Oxide von 3-Cephem-Verbindungen kann in an sich bekannter Weise durch Behandeln mit einem Reduktionsmittels, wenn notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid,-acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwe det werden; reduzierende trivalente anorganische oder organische Phosphorverbindungen, wie Phosphine, ferner Ester, Amide und Halogenide der phosphonigen, phosphinigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes Niederalkyl, Phenyl oder Phenylniederalkyl darstellen, wie z.B. Triphenylphosphin, Tri-n-butylphosphin, Diphenylphosphonigsäuremethylester, Diphenylchlorphosphin, Phenyldichlorphosphin, Benzolphosphonigsäuredimethylester, Butanphosphonigsäuremethylester, Phosphorsäuretriphenylester, rhosphorigsäuretrimethylester, Phosphortrichlorid, Phosphortribromid, etc.; reduzierende Ralogensilanverbindungen, die mindestens ein an das Siliciumatom gebundenes' Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, .Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefel enthaltende Silane, wie 1,3-Dithia-2,4,-disilacyälobutane oder 1,3,5-Trithia-2,4,6-trisilacyclohexane, deren Siliciumatome durch Kohlenwasserstoffreste, wie insbesondere Niederalkyl substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethyleniminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylenpyrro-Iidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor-oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphorcrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, Propansulton, 1,3-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureestär oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

### Verfahren c) (Bildung der gegebenenfalls substituierten Hydroxyiminogruppe):

In einem Ausgangsmaterial der Formel V, worin die 4-Carboxylgruppe sowie weitere funktionelle Gruppen gegebenenfalls geschützt sind, kann die nicht zur Amidgruppierung gehörende Oxogruppe in der 7β-Acylamidogruppe durch Behandeln mit einem Hydroxylaminderivat der Formel VI in eine gegebenenfalls 0-substituierte Hydroxyiminogruppe übergeführt werden. Die Reaktion eines Ausgangsmaterials der Formel V mit einem Hydroxylaminderivat der Formel VI wird in üblicher Weise durchgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, z.B. in einem Temperaturbereich von etwa -20°C bis etwa +100°C, bevorzugt von etwa 0°C bis etwa 30°C, gegebenenfalls in einer Inertgas-, wie Stickstoffatmosphäre, umsetzt. Die Hydroxylaminverbindung der Formel VI kann auch in situ, z.B. aus einem ihrer Salze, wie einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyldiisopropylamin, oder einer heterocyclischen tertiären Base, wie Pyridin, in Freiheit gesetzt werden.

### Verfahren d) (Bildung der unsubstituierten Hydroxyiminogruppe) :

In einem Ausgangsmaterial der Formel VII sind die funktionellen Gruppen vorzugsweise geschützt. Geeignete Nitrosierungsmittel sind salpetrige Säure und davon abgeleitete Derivate, wie Nitrosylhalogenide, z.B. Nitrosylchlorid oder Nitrosylbromid, Salze der salpetrigen Säure, wie Alkalimetallsalze, z.B. Natrium- oder Kaliumnitrit, oder insbesondere Ester der salpetrigen Säure, wie entsprechende Niederalkylester, z.B. Butyl-, Pentyl- oder insbesondere Isoamylnitrit. Verwendet man als Nitrosierungsmittel ein Salz der salpetrigen Säure, so wird die Reaktion vorzugsweise in Gegenwart einer starken anorganischen oder organischen Säure, z.B. Chlorwasserstoffsäure, Schwefelsäure, Ameisensäure oder Essigsäure, durchgeführt. Bei Verwendung eines Esters der salpetrigen Säure wird die Reaktion vorzugsweise in Gegenwart einer starken Base, wie eines Alkalimetallalkanolates, durchgeführt.

Die Nitrosierung wird in einem geeigneten Lösungsmittel, wie Wasser, einer Carbonsäure, z.B. Essigsäure, einem Niederalkanol, z.B. Methanol oder Aethanol, einem Aether, z.B. Dioxan oder Tetrahydrofuran, oder einem Kohlenwasserstoff, z.B. Hexan oder Benzol, oder in Mischungen davon, wenn notwendig unter Kühlen oder Erwärmen, insbesondere in einem Temperaturbereich von etwa -15°C bis etwa Raumtemperatur, und/oder in einer Inertgasatmosphäre durchgeführt.

### Verfahren e) (Bildung der Aminothiadiazolylgruppe):

In einem Ausgangsmaterial der Formel VIII ist Xₗ als Halogen beispielsweise Chlor oder Jod, bevorzugt jedoch Brom. Geeignete Salze der Rhodanwasserstoffsäure sind beispielsweise entsprechende Alkali-, Erdalkali- oder auch Schwermetallsalze, z.B. das Natrium-, Kalium-, Magnesium- oder Bleirhodanid, oder auch Ammoniumrhodanide, worin die Ammoniumgruppe von Ammoniak oder von organischen Stickstoffbasen abgeleitet ist, z.B. Ammonium- oder Mono-, Di-, Tri- oder Tetraniederalkylammoniumrhodanid, worin Niederalkyl z.B. Methyl, Aethyl oder Isopropyl bedeutet.

Geeignete Isorhodanwasserstoffsäureester sind beispielsweise Isorhodanwasserstdffsäureniederalkylester, die auch Niederalkylisothiocyanate genannt werden, wie Methyl-, Aethyl-, Propyl- oder Butylisothiocyanat.

Die Kondensation von Verbindungen der Formel VIII mit einem Salz der Rhodanwasserstoffsäure oder mit Dirhodan ergibt Verbindungen der Formel I, worin Am eine primäre Aminogruppe ist, während die Kondensation mit einem Isorhodanwasserstoffsäureester eine Verbindung der Formel I ergibt, worin Am eine freie sekundäre Aminogruppe, beispielsweise eine Niederalkylaminogruppe ist.

Die Reaktion wird bevorzugt in einem inerten Lösungsmittel, wie einem Niederalkanol, z.B. Methanol, Aethanol, Propanol oder Isopropanol, einem Aether, wie Diäthyläther, Dioxan oder Tetrahydrofuran, einem Amid, wie Dimethylformamid, einem aliphatischen oder aromatischen Kohlenwasserstoff, wie Hexan, Benzol oder Toluol, oder auch in Mischung davon, und, wenn notwendig, unter Kühlen oder Erwärmen, d.h. bei Temperaturen zwischen etwa -80°C bis etwa +100°C, bevorzugt zwischen etwa -20°C bis etwa +20°C, und/oder in einer Inertgasatmosphäre durchgeführt.

Ein Ausgangsmaterial der Formel VIII, worin X₁ Halogen ist, kann auch in situ hergestellt werden, gegebenenfalls in Gegenwart des Rhodanwasserstoffsalzes, beispielsweise indem man eine Verbindung der Formel VIII, worin X₁ Wasserstoff ist, in einem der genannten Lösungsmittel mit einem Hypohalogenit, z.B. Natrium- oder Kaliumhypobromit, oder mit einem Halogen und einer Alkalimetallbase, wie einem Hydroxid, Carbonat oder Niederalkanolat, z.B. mit Brom und Natriummethylat oder Kaliumcarbonat, behandelt. Die Halogenierung findet bevorzugt unter Kühlen, z.B. bei einer Temperatur von etwa -15°C bis etwa 0°C statt.

Eine Verbindung der Formel VIII, worin X Hydroxy ist, kann ebenfalls in sitü hergestellt werden, beispielsweise indem man eine Verbindung der Formel VIII,worin X Wasserstoff ist, mit einem Oxidationsmittel, wie Wasserstoffperoxid, behandelt.

Die Reaktion eines Ausgangsmaterials der Formel VIII, worin X Wasserstoff ist, mit Dirhodan wird vorzugsweise ebenfalls in einem der genannten inerten Lösungsmittel und unter ähnlichen Reaktionsbedingungen durchgeführt. Gegebenenfalls kann auch ein Säureadditionssalz einer Verbindung der Formel VIII eingesetzt werden, das in situ in die freie Verbindung übergeführt wird, oder das Dirhodan kann in situ gebildet werden, z.B. aus einem Rhodanid, z.B. Bleirhodanid, und einem Halogen, z.B. Brom.

### Verfahren f) (Einführung einer Ammoniogruppe):

In einem Ausgangsmaterial der Formel IX, worin die 4-Carboxylgruppe sowie weitere funktionelle Gruppen gegebenenfalls geschützt sind, stellt die Gruppe X₂ vorzugsweise eine veresterte Hydroxygruppe dar, insbesondere eine durch eine organische Säure, wie Niederalkancarbonsäure, z-B. Ameisen- oder Essigsäure, ferner eine anorganische Säure, wie Halogenwasserstoffsäure, z.B. Brom- oder Jodwasserstoffsäure, veresterte Hydroxygruppe.

Die Reaktion mit einer dem Rest R₁ entsprechenden tertiären organischen Base, insbesondere einem gegebenenfalls substituierten Pyridin, wird unter neutralen oder schwach sauren Bedingungen in einem pH-Bereich von 3-8, vorzugsweise bei etwa pH 6,5, in Gegenwart von Wasser und gegebenenfalls in einem mit Wasser mischbaren organischen Lösungsmittel vorgenommen. Die schwach sauren Bedingungen können durch Zugabe einer geeigneten organischen oder anorganischen Säure, beispielsweise Essigsäure, Chlorwasserstoffsäure, Phosphorsäure oder auch Schwefelsäure eingestellt werden. Als organische Lösungsmittel können beispielsweise mit Wasser mischbare Lösungsmittel, wie Niederalkanole, z.B. Methanol oder Aathanol, Niederalkanone, z.B. Aceton, Niederalkancarbonsäureamide, z.B. Dimethylformamid, oder Niederalkancarbonsäurenitrile, z.B. Acetonitril, verwendet werden. Ferner können, z.B. zur Erhöhung der Ausbeuten dem Reaktionsgemisch geeignete Salze, wie Alkalimetall-,"z.B. Natrium-und insbesondere Kaliumsalze, von anorganischen Säuren, wie Halogenwasserstoffsäuren, z.B. Chlorwasserstoff- und insbesondere Jodwasserstoffsäuren, sowie der Thiocyansäure, oder von organischen Säuren, wie Niederalkancarbonsäuren, z.B. Essigsäure, insbesondere Kaiiumjodid und Kaliumthiocyanat, zugesetzt werden. Auch Salze von geeigneten Anionenaustauschern mit Säuren, z.B. Essigsäure, z.B. flüssige Ionenaustauscher in Salzform, z.B. Amberlite LA-1 (flüssige sekundäre Amine mit einem Molekulargewicht von 351-393; Oel-löslich und Wasser-unlöslich; mAeq./g = 2,5-2,7, z.B. in Acetatform), können für diesen Zweck verwendet werden.

Ammoniogruppen R₁ können vorteilhafterweise unter Verwendung eines Zwischenproduktes der Formel IX, worin X₂ für eine substituierte, insbesondere für eine aromatische substituierte Carbonylthiogruppe und in erster Linie für die Benzoylthiogruppe steht, eingeführt werden. Ein solches Zwischenprodukt kann man z.B. durch Umsetzen eines Ausgangsmaterials der Formel IX, worin X₂ eine veresterte Hydroxygruppe, insbesondere Niederalkanoyloxy, z.B. Acetyloxy, bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten. Das Zwischenprodukt wird dann mit der tertiären Aminbase, insbesondere einer entsprechenden heterocyclischen Base, wie einem gegebenenfalls substituierten Pyridin, umgesetzt, wobei man die gewünschte Ammoniumverbindung erhält. Die Reaktion wird üblicherweise in Gegenwart eines geeigneten Entschwefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatmosphäre, durchgeführt.

### Verfahren g) (Austausch von Y gegen Amine AmH):

In einer Verbindung der Formel X ist Y Brom, Jod oder bevorzugt Chlor.

Die Amine Am-H werden in freier Form, d.h. als Ammoniak, Niederalkylamin, wie Methyl-, Aethyl-, Propyl- oder Butylamin, oder Diniederalkylamin, wie Dimethyl-, Diäthyl-, Dipropyl-, Dibutyl-, Methyläthyl-, Aethylpropyl-, Methylbutyl-, Propylbutylamin, oder dergleichen, oder als Metallamide davon, beispielsweise als Alkali- metallamide,wie Lithium-, Natrium- oder Kaliumamide, eingesetzt.

Die Austauschreaktion findet bevorzugt in einem inerten organischen Lösungsmittel, wie Benzol, Toluol, Dimethylformamid, Tetrahydrofuran, oder dergleichen, oder, beim Austausch von Y gegen NH₂, auch in flüssigem Ammoniak statt, wobei die Säure H-Y abfangende Basen, wie tertiäre Amine, z.B. Aethyldiisopropylamin, Pyridin, oder dergleichen, zugefügt werden können.

Die Reaktion erfolgt, je nach Reaktionsfähigkeit, bei Zimmertemperatur oder unter Abkühlen oder Erwärmen, zwischen etwa -70°C und etwa +100°C, bevorzugt zwischen etwa -70°C und etwa +50°C.

Die erfindungsgemäss erhältlichen Verbindungen der Formel I können in an sich bekannter Weise in andere Verbindungen der Formel I übergeführt werden.

Substituierung einer Hydroxyiminogruppe: In einer Verbindung der Formel I, worin die 4-Carboxylgruppe sowie weitere funktionelle Gruppen, insbesondere solche funktionelle Gruppen, die in analoger Weise wie die Hydroxyiminogruppe substituiert werden können, vorzugsweise geschützt sind, kann die Hydroxygruppe des unsubstituierten Hydroxyiminorestes in an sich bekannter Weise veräthert werden.

Geeignete Verätherungsmittel sind z.B. Diazoniederalkane, z.B. Diazomethan, Ester von Alkoholen der Formel R₂-OH, worin R₂ gegebenenfalls substituiertes Niederalkyl oder Cycloalkyl darstellt, mit starken anorganischen oder organischen Säuren, wie Niederalkyl- oder Cycloalkylhalogenide, z.B. -chloride, -bromide- oder -iodide, oder Diniederalkylsulfate, ferner Fluorsulfonsäureniederalkylester, oder gegebenenfalls Halogensubstituierte Methansulfonsäureniederalkylester, sowie geeignete Acetale, z.B. gem-Di-niederalkoayniederalkane, Tri-R₂-oxoniumsalze (sogenannte Meerweinsalze), Di-R₂-O-Carbenium- salze oder Di-Rz-Haloniumsalze, worin R.2 gegebenenfalls substituiertes Niederalkyl oder Cycloalkyl darstellt, wie Triniederalkyloxoniumsalze, sowie Diniederalkoxycarbenium- oder Diniederalkylhaloniumsalze, insbesondere die entsprechenden Salze mit komplexen, fluorhaltigen Säuren, oder 1-R₂-3-Aryltriazenverbindungen, worin R₂ gegebenenfalls substituiertes Niederalkyl oder Cycloalkyl und Aryl vorzugsweise einen gegebenenfalls substituierten Phenylrest bedeutet. Diese Verätheruagsreagenzien werden in an sich bekannter Weise, z.B. wie oben beschrieben, verwendet, üblicherweise in einem geeigneten Lösungsmittel oder Lösungsmittelgemisch, wenn notwendig, in Gegenwart eines Kondensationsmittels, wie einer Base, unter Kühlen oder Erwärmen, in einem geschlossenen Gefäss unter Druck und/oder in einer Inertgas-, z.B. Stickstoffatmosphäre.

Die Einführung einer Carbamoylgruppe -C(=O)-NHR- anstelle des Wasserstoffatoms geschieht auf an sich bekannte Weise durch Carbamoylierung. Zu diesem Zeck wird eine Verbindung der Formel I, worin R₂ Wasserstoff bedeutet und alle reaktionsfähigen Gruppen, welche nicht carbamoylierrwerden sollen oder die Reagenzien verbrauchen oder zerstören können, in geschützter Form vorliegen, gegebenenfalls stufenweise mit einem Carbamoylierungsmittel behandelt. Geeignete Carbamoylierungsmittel sind reaktionsfähige Derivate von Carbaminsäuren R-NH-(=O)OH, insbesondere Isocyanate R-N=C=O oder gemischte Anhydride, z.B. Carbaminsäurechloride R-NH-C(=O)Cl, die auch in situ aus Phosgen und einem Amin R-NH₂ hergestellt werden können.

Der Umsatz mit Isocyanaten erfolgt in einem der üblichen inerten Lösungsmittel, beispielsweise einem gegebenenfalls halogenierten Kohlenwasserstoff, z.B. Benzol, Xylol oder Methylenchlorid, einem Aether, z.B. Diäthyläther, Dioxan oder Tetrahydrofuran, oder einem Ester, z.B. Essigsäureäthylester, gegebenenfalls in Gegenwart eines basischen Katalysators, beispielsweise eines tertiären Amins, z.B. Triäthylamin oder Pyridin, bei Raumtemperatur oder etwas erniedrigter oder erhöhter Temperatur, etwa zwischen -20°C und +50°C.

Zum Umsatz mit einem gemischten Anhydrid der Carbaminsäure wird die Alkoholgruppe vorzugsweise zunächst metallisiert, beispielsweise mit einem Alkalimetallhydrid, z.B. Natrium- oder Lithiumhydrid. Die an der Hydroxygruppe metallierte Verbindung der Formel I kann auch zunächst mit Phosgen und anschliessend mit dem Amin R-NH₂ behandelt werden und auf diese Art stufenweise carbamoyliert werden. Diese Reaktionen finden ebenfalls in einem inerten Lösungsmittel und bei den oben angegebenen Temperaturen statt.

Substituierung einer Aminogruppe: Eine gegebenenfalls monosubstituierte Aminogruppe Am in einer erfindungsgemäss erhältlichen Verbindung der Formel I, worin die anderen vorhandenen funktionellen Gruppen, insbesondere solche, die analog wie eine Aminogruppe substituiert werden können, vorzugsweise geschützt sind, kann in an sich bekannter Weise, z.B. durch Behandeln mit einem reaktionsfähigen Ester eines Niederalkanols, wie einem Niederalkylhalogenid, z.B. -chlorid, -bromid- oder -jodid, vorzugsweise in Gegenwart eines Lösungsmittel, oder, unter Einführung von Methyl, mit Formaldehyd in Gegenwart von Ameisensäure substituiert werden. Ferner kann man eine gegebenenfalls monosubstituierte Aminogruppe Am mit einem geeigneten Derivat einer Niederalkancarbonsäure, wie einem entsprechenden Halogenid, z.B. Chlorid, acylieren, und in einer so erhältlichen Niederalkanoylamino-Verbindung die Carbonylgruppe, z.B. durch Behandeln mit einem geeigneten Hydridreduktionsreagens, wie Diboran, zu einer Methylengruppe reduzieren. Die Substitutionsreaktion wird in an sich bekannter Weise, bevorzugt in Gegenwart eines Lösungsmittels, und wenn notwendig, unter Kühlen oder Erwärmen und/oder in einer Inertgasatmosphäre durchgeführt.

Bildung des 1-Oxids: Eine Verbindung der Formel I, worin n für 0 steht, kann durch Oxidation, z.B. nach einer der unter Verfahren b) beschriebenen Oxidationsmethoden, in eine Verbindung der Formel I, worin n für 1 steht, überführt werden.

Reduktion des 1-Oxids: Eine Verbindung der Formel I, worin n für 1 steht, kann durch Reduktion, z.B. nach einer der unter Verfahren b) beschriebenen Reduktionsmethoden, in eine Verbindung der Formel I, worin n für 0 steht, überführt werden.

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy-und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

So kann man tart.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mi,leinem Alkalimetall-, z.B. Natriumdithionit, oder mit einem reduzierenden Metall, z.B. Zink, oder Metallsalz, wie einem Chram-II-salz, z.B. Chram-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, kann man, wie oben beschrieben, auch 2-Halogenniederalkoxycarbonyl, gegebenenfalls nach Umwandlung einer 2-Bromniederalkoxycarbonylgruppe in eine entsprechende 2-Jodniederalkoxycarbonylgruppe, oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe, z.B. Am, setzt man in an sich bekannter und je nach Art der Schutzgruppen in verschiedenartiger Weise, vorzugsweise mittels Solvolyse oder Reduktion, frei. 2-Halogenniederalkoxycarbonylamino gegebenenfalls nach Umwandlung einer 2-Brom-niederalkoxycarbonylaminogruppe in eine 2-Jod-niederalkoxycarbonylaminogruppe, Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-l-en-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloraeetyl, geschützte Aminogruppe kann durch Behandeln mit Thioharnstoff in Gegenwart einer Base, oder mit einem Thiolatsalz, wie einem Alkalimetallthiolat, der Thioharnstoffs und anschliessende Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosp hon- säure.oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silyl-oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, während eine durch tart.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator,' wie einem Palladium/Kohle/Katalysator.

Salzbildung: Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der a-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe enthalten, können z.B. durch Neutralisieren von Salzen, wie Säureadditionssalzen, auf den isoelektrischen Punkt, z.B. mit schwachen" Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe: Die im Verfahren zur Herstellung der Verbindungen der vorliegenden Erfindung verwendeten Ausgangsstoffe sind bekannt oder, falls neu, können sie in an sich bekannter Weise hergestellt werden.

Die Ausgangsverbindungen vom Typ der Formel II und III, sowie entsprechende Verbindungen mit geschützten funktionellen Gruppen sind bekannt oder können auf an sich bekannte Weise hergestellt werden.

Die Herstellungsweise von Ausgangsstoffen der Formel III wird anhand des folgenden Reaktionsschemas zur Herstellung einer 2-(5-Amino-1,2,4-thiadiazol-3-yl)-2-methoxyimino-essigsäureverbindung illustriert:

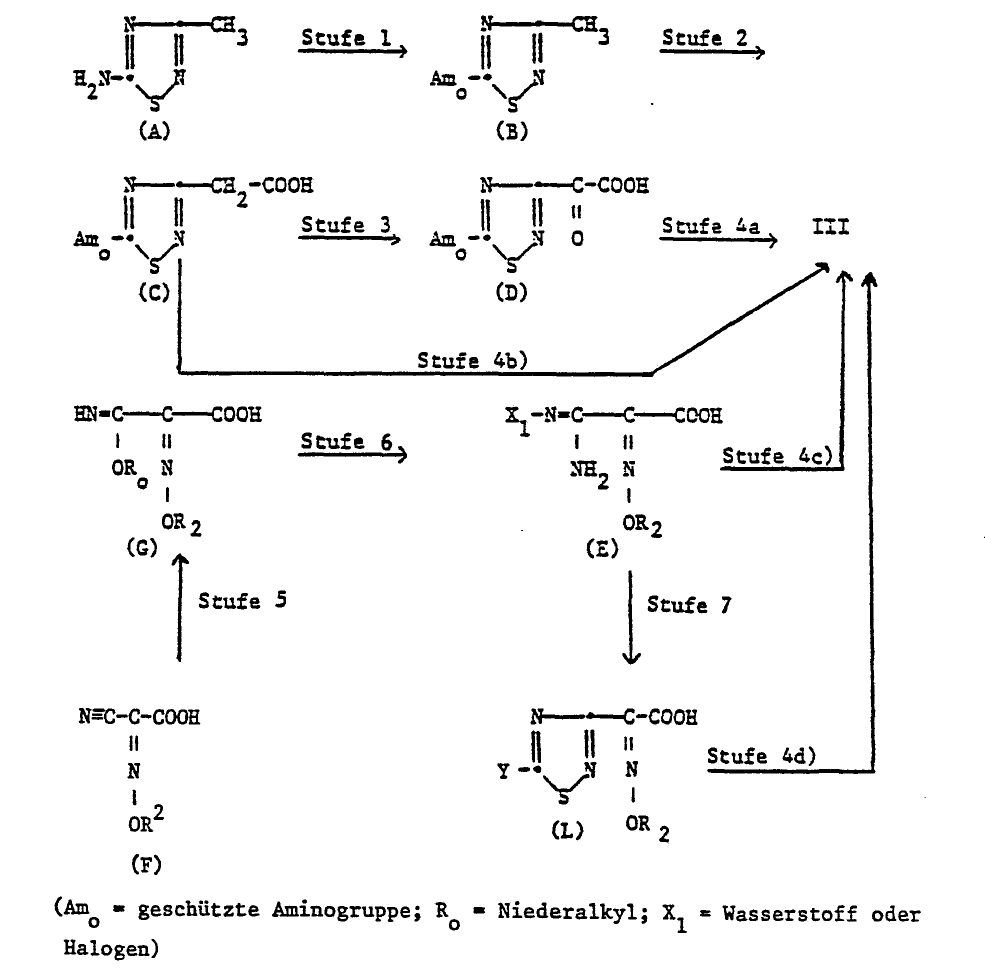

Ausgangsstoffe der Formel IV können u.a. als Nebenprodukt bei der Herstellung von Verbindungen vom Typ der Formel I erhalten werden, z.B. wenn diese unter basischen Bedingungen gebildet werden. Sie brauchen nicht in reiner Form vorzuliegen, sondern können z.B. auch im Gemisch mit entsprechenden Verbindungen der Formel I eingesetzt werden.

Verbindungen der Formel V können z.B. durch Acylieren von Verbindungen der Formel II mit Verbindungen der Formel D oder einem reaktionsfähigen funktionellen Derivat davon, z.B. nach einer der unter Verfahren a) beschriebenen Methoden, erhalten werden.

Verbindungen der Formel VII können z.B. durch Acylieren von Verbindungen der Formel II mit einem den Acylrest einer Säure der Formel C einführenden Acylierungsmittel, z.B. nach einer der unter Verfahren a) beschriebenen Methoden, erhalten werden.

Verbindungen der Formel VIII können z.B. durch Acylieren von Verbindungen der Formel II mit einem den Acylrest einer Säure der Formel E einführenden Acylierungsmittel, z.B. nach einer der unter Verfahren a) beschriebenen Methoden erhalten werden. Ferner kann man, analog den Stufen 6 und 7 im Reaktionsschema zur Herstellung von Ausgangsstoffen der Formel III, ausgehend von Verbindungen der Formel 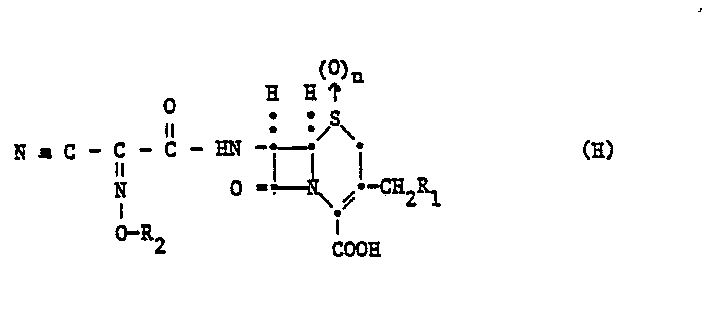 über Verbindungen der Formel 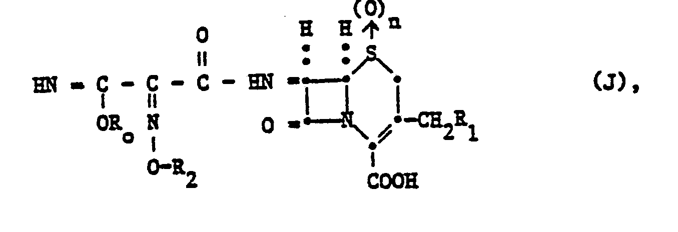 zu den gewünschten Ausgangsstoffen der Formel VIII gelangen. Die Verbindungen der Formeln H und J können ihrerseits durch Acylierung von Verbindungen der Formel II mit einem den Acylrest einer Säure der Formel F bzw. G einführenden Acylierungsmittel, z.B. nach einer der unter Verfahren a) beschriebenen Methoden, erhalten werden.

Ausgangsstoffe der Formel IX kann man z.B. erhalten, indem man von den entsprechenden 7β-Amino-3-(X₂-CH₂)-3-cephem4-carbonsäure Verbindungen ausgeht und die Aminogruppe z.B. durch Behandeln mit einem den Acylrest einer Säure der Formel III einführenden Acylierungsmittel, z.B. nach einer der unter Verfahren a) beschriebenen Methoden, acyliert.

Verbindungen der Formel X können auf verschiedene Weise hergestellt werden, beispielsweise analog den Verfahren a) oder b) oder der Stufe 7, insbesondere indem man, analog Verfahren a), in einer Verbindung der Formel II, worin n, R₁ und R₂ die angegebenen Bedeutungen haben und worin die 7ß-Aminogruppe gegebenenfalls durch eine die Acylierung erlaubende Gruppe substituiert ist, die 7ß-Aminogruppe durch Behandeln mit einem den Acylrest einer Carbonsäure der Formel L einführenden Acylierungsmittel acyliert, oder indem man, analog Stufe 7, eine Verbindung der Formel VIII, worin X₁ Wasserstoff bedeutet und n, R₁ und R₂ die angegebenen Bedeutungen haben, oder ein Salz davon, mit einem Perhalogenmethylmercaptan der Formel Y₃C-S-Y umsetzt, und gewünschtenfalls die bei der Herstellung der Verbindungen der Formel I genannten nachträglichen Operationen durchführt.

Neue Ausgangsverbindungen, sowie Zwischenprodukte und Verfahren zu ihrer Herstellung sind ebenfalls Gegenstand der vorliegenden Erfindung.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen,, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von parenteral, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz..allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa l% bis etwa 50%, Lyophilisate bis zu 100% des Aktivstoffes.

Verbindungen der Formel I können auch oral, z.B. in Form von Kapseln, verabreicht werden. Diese enthalten den Wirkstoff, gegebenenfalls zusammen mit geeigneten Trägerstoffen, in Form eines Granulats und zwar in Dosen von etwa 0,2 bis etwa 0,5 g pro Dosiseinheit.

Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgende Abkürzungen verwendet:
BOC: tert.-Butyloxycarbonyl
F.: Schmelzpunkt

Dünnschichtchromatographie: Opti-UPC₁₂-Platten der Fa. Antec sind mit Silicagel beschichtete Glasplatten, wobei das Silicagel mit Dodecyltrichlorsilan behandelt wurde (zur Chromatographie mit umgekehrter Phase).

### Beispiel 1:

### a) 7β-[2-(5-Amino-1,2,4-thiadiazol-3-yl)-2-Z-methoxyimino-acetamido]-3-(4-carboxy-pyridiniomethyl)-3-cephem-4-carboxylat

Eine Lösung von 0,478 g (1,0 mMol) Natrium-7ß-[2-(5-amino-1,2,4-thiadiazol-3-yl)-2-Z-methoxyiminoacetamido]-3-acetoxymethyl-3-cephem-4-carboxylat in 1 ml Wasser wird zu einer 80° warmen Lösung von 0,17 g (1,39 mMol) Isonicotinsäure und 1,71 g Natriumjodid in 0,4 ml Wasser gegeben und während 2 Stunden bei 70° gerührt. Nach Abkühlen auf Raumtemperatur wird das Reaktionsgemisch auf 100 ml kaltes Aceton gegossen, der gebildete Niederschlag filtriert und getrocknet. Nach chromatographischer Reinigung des Rohproduktes (Silicagel "Opti UPC₁₂", Wasser) erhält man die einheitliche Titelverbindung mit einem Rf-Wert von 0,25 (UFC₁₂-Platten, Wasser/Acetonitril 6:1); IR-Spektrum (Nujol): charakteristische Absorptionsbanden bei 3,05; 5,70; 6,25; 9,60 µ.

Das Ausgangsmaterial kann wie folgt hergestellt werden:

### b) Natrium-7β-[2-(5-Amino-1,2,4-thiadiazol-3-yl)-2-Z-methoxyimino- acetamido]-3-acetoxymethyl-3-cephem-4-carboxylat

7β-[2-(5-Amino-1,2,4-thiadiazol-3-yl)-2-Z-methoxyiminoacet- amido]-3-acetoxymethyl-3-cephem-4-carbonsäure wird in Methanolhaltigem Wasser (10 ml Methanol/500 ml Wasser) gelöst und die wässrige Phase mit wässriger Natriumbicarbonatlösung auf pH 6,8 gestellt. Die Lösung wird lyophilisiert, und die erhaltenen Natriumsalze werden mittels Chromatographie an Silicagel "Opti UPC₁₂ " (Fa. Antec) mit Wasser/Acetonitril 6:1 gereinigt. Man erhält die einheitliche Titelverbindung vom Rf-Wert 0,30 (UPC₁₂-Platten, Wasser/Acetonitril 6:1), F. ab 150° (Zers.); UV-Spektrum (Methanol): λₘₐₓ (ε): 234 (12240) nm; IR-Spektrum (Nujol): charakteristische Absorptionsbanden bei 3.00; 5.65; 5,95; 6.20; 6.52; 8.10; 9,60 µ.

### Beispiel 2:

### a) 7β-[2-(5-Amino-1,2,4-thiadiazol-3-yl)-2-Z-2-carboxyprop-2-yloxy- iminoacetamido]-3-(3-carboxymethylpyridiniomethyl)-3-cephem- 4-carboxylat

Zu einer auf 80°C vorgewärmten Lösung von 6,6 g (44 mMol) Natriumjodid und 0,58 g (4,22 mMol) Pyridyl-3-essigsäure in 1,6 ml dest. Wasser gibt man eine getrennt davon zubereitete Lösung von 2,28 g (3,84 mMol) Dinatrium-7-ß-[2-(5-amino-l,2,4-thiadiazol-3-yl)-2-Z-2-carboxyprop-2-yloxyiminoacetamido]- 3-acetoxymethyl-3-cephem-4-carboxylat in 4 mldest. Wasser. Nach Zugabe von 0,23 ml Essigsäure (100%) wird 3 Stunden lang bei 70°C gerührt. Die Reaktionslösung wird unter Kühlung auf -20° in 150 ml Aceton eingetragen. Der gebildete Niederschlag wird abfiltriert, in 40 ml dest. Wasser gelöst und lyophilisiert. Nach chromatographischer Reinigung des Rohprodukts (Silicagel "Opti UPC₁₂", Wasser) erhält man die einheitliche Titelverbindung mit einem Rf-Wert von 0,19 (UPC₁₂-Platten, Wasser). IR-Spektrum: 2.95; 5.64; 6.27 und 6.52 µ.

Das Ausgangsmaterial kann wie folgt hergestellt werden:

### b) 2-(5-tert.-Butoxycarbonylamino-1,2,4-thiadiazol-3-yl)-essig- säuremethylester

Eine Mischung von 30,0 g (115,0 mMoI)2-(5-tert.-Butoxycar- bonylamino-l,2,4-thiadiazol-3-yl)-essigsäure in 350 ml Methylenchlorid wird nacheinander mit 26,16 g N,N'-Dicyclohexylcarbodiimid, 5,13 ml abs. Methanol und 1,71 g 4-Pyrrolidinopyridin versetzt und während 3 Stunden bei Raumtemperatur gerührt. Nach Zugabe von weiteren 0,5 ml abs. Methanol und 2,6 g N,N'-Dicyclohexylcarbodiimid wird für weitere 60 Minuten gerührt. Der Niederschlag wird abgenutscht, das Filtrat nacheinander mit wässriger Natriumbicarbonatlösung, Wasser und wässriger Natriumchloridlösung gewaschen,ueber Magnesiumsulfat getrocknet und im Vakuum eingeengt. Der Rückstand wird mit Diäthyläther verrieben. Man erhält die einheitliche Titelverbindung vom Rf-Wert 0,50 (Silicagel, Toluol:Aethylacetat 1:1); IR-Spektrum (CH₂C1₂): charakteristische Absorptionsbanden bei 2.90; 5,75; 5,85; 6.50; 8.70 µ.

### c) 2-(5-tert.-Butoxycarbonylamino-1,2,4-thiadiazol-3-yl)-2-oxoessig- säure-methylester

Eine Suspension von 20,0 g (73,18mMol) 2-(5-tert.-Butoxy- carbonylamino-1,2,4-thiadiazol-3-yl)essigsäuremethylester und 22,0 g Selendioxid in 330 ml abs. Dioxan wird 3 Stunden bei 95° gerührt. Nach Filtrieren des Reaktionsgemisches wird das Filtrat im Vakuum eingeengt. Der Rückstand wird mit Aethylacetat verdünnt, nacheinander mit Wasser, wässriger Natriumbicarbonatlösung und wässriger Natriumchloridlösung gewaschen, über Magnesiumsulfat getrocknet und eingeengt. Man erhält die Titelverbindung in Form eines Schaumes vom Rf-Wert 0,35 (Silicagel, Toluol:Aethylacetat 1:1); IR-Spektrum (CH₂Cl₂): charakteristische Absorptionsbanden bei 2.95; 5.75; 5.85; 6.50 µ.

### d) 2-(5-tert.-Butozycarbonylamino-1,2,4-thiadiazol-3-yl)-2-hydroxy- ⁱminoessigsäure-methylester

Eine Suspension von 21,8 g (75,88 mMol) 2-(5-tert.-Butoxy- carbonylamino-1,2,4-thiadiazol-3-yl)-2-oxoessigsäuremethylester, 7,48 g Hydroxylaminhydrochlorid und 9,8 ml Pyridin in 300 ml 95%-igem Aethanol wird 3 Stunden bei Raumtemperatur gerührt. Das Reaktionsgemisch wird im Vakuum eingeengt. Der Rückstand wird in Aethylacetat gelöst und die Lösung nacheinander mit verdünnter Salzsäure, Wasser und wässriger Natriumchloridlösung extrahiert, über Magnesiumsulfat getrocknet und im Vakuum eingeengt. Der Rückstand wird aus Diäthyläther kristallisiert. Man erhält die Titelverbindung vom Rf-Wert 0,30 (Silicagel, Chloroform:Methanol 95:5); F. 131-132°; IR-Spektrum (CH₂Cl₂): charakteristische Absorptionsbanden bei 2.85; 5.75; 5.85; 6.50; 8.70; 8.90 µ.

### e) 2-(5-tert.-Butoxycarbonylamino-1,2,4-thiadiazol-3-yl)-2-Z-(2-tert.-butoxycarbonylprop-2-yloxyimino)essigsäuremethylester

Eine Lösung von 1,4 g (6,27 mMol) 2-Bromisobuttersäure-tert.-- butylester und 1,84 g (14,03 mMol) fein pulverisiertem Kaliumcarbonat in 12,50 ml Dimethylsulfoxid wird bei Zimmertemperatur unter Stick- - -stoff mit einer Lösung von 1,70 g (5,62 mMol) 2-(5-tert.-Butoxycarbo- nylamino-1,2,4-thiadiazol-3-yl)-2-hydroxyiminoessigsäure-methylester in 7,5 ml Dimethylsulfoxid versetzt und 14 Stunden bei dieser Temperatur gerührt. Das Reaktionsgemisch wird bei 30° im Hochvakuum zur Trockne eingedampft. Der Rückstand wird in 200 ml Essigsäureäthylester aufgenommen und nacheinander mit Wasser, verdünnter Salzsäure (1 N), Wasser und gesättigter Natriumchloridlösung gewaschen, über Magnesiumsulfat getrocknet und im Vakuum zur Trockne eingedampft. Der Rückstand wird aus ca. 5 ml Aether kristallisiert und ergibt die Titelverbindung vom Schmelzpunkt 142-144°.

### f) 2-(5-tert.-Butoxycarbonylamino-1,2,4-thiadiazol-3-yl)-2-Z-(2-tert.-butoxycarbonylprop-2-yloxyimino)essigsäure

Man lässt eine Lösung von 11 g (22,55 mMol) 2-(5-tert.-Butoxy- carbonylamino-1,2,4-thiadiazol-3-yl)-2-Z-.(2-tert.-butoxycarbonylprop-2-yloxyimino)-essigsäuremethylester in 290 ml Methanol und 96 ml 2 N Natronlauge bei einer Temperatur von 50° 6 1/2 Stunden rühren. Das Reaktionsgemisch wird bei 35° am Hochvakuum zur Trockne eingedampft. Der Rückstand wird in 200 ml Wasser (destilliert) aufgenommen und einmal mit 200 ml Essigsäureäthylester extrahiert. Die wässrige Phase wird mit 200 ml Essigsäureäthylester überschichtet und mit konz. Salzsäure sauer gestellt. Die Essigsäureäthylesterphase wird nacheinander mit Wasser und gesässtigter Natriumchloridlösung gewaschen, über Magnesiumsulfat getrocknet und im Vakuum eingeengt, wobei die Titelverbindung kristallin anfällt; DC-Rf-Wert: 0,2 (Silicagel, Chloroform/Methanol 4:1); IR-Spektrum (Dioxan): charakteristische Banden bei 5.81; 5.74; 3.3 µ; F: 180-183°.

### g) 7β-[2-(5-tert.-Butoxycarbonylamino-1,2,4-thiadiazol-3-yl)-2-Z-(2-tert.-butoxycabonylprop-2-yloxyimino)-acetamido]-3-acetoxymethyl-3-cephem-4-carbonsäure

Eine Suspension von 15 ml Methylenchlorid abs., 0,3 ml Dimethylformamid und 0,33 ml (7,84 mMol) Oxalsäuredichlorid wird bei -10° 30 Minuten unter Stickstoff gerührt. Dann wird 1,3 g (3,01 mMol) . 2-(5-tert.-Butoxycarbonylamino-1,2,4-thiadiazol-3-yl)-2-Z-(2-tert.-butoxycarbonylprop-2-yloxyimino)-essigsäure zur Suspension gegeben. Die entstehende klare Lösung wird 30 Minuten bei 0-5° unter Stickstoff gerührt. Zu dieser Lösung tropft man eine frisch hergestellte Lösung von 0,87 g (3,19 mMol) 7ß-Aminocephalosporansäure in 18 mIMethylenchlorid abs.und 3 ml N,0-Bis-(trimethylsilyl)-acetamid und rührt bei einer Temperatur von 0-5° unter Stickstoff 16 Stunden. Die Reaktionslösung wird nacheinander mit Wasser und gesättigter Natriumchloridlösung gewaschen, über Magnesiumsulfat getrocknet und am Vakuum zum trockenen Schlamm eingedampft. Man erhält die Titelverbindung vom Rf-Wert 0,43 (Silicagel, Essigester/Essigsäure 9:1); IR-Spektrum (Nujol): charakteristische Absorptionsbanden bei 5.6; 5.82; 6.04 µ.

### h) Dinatrium-7β-[2-(5-Amino-1,2,4-thiadiazol-3-yl)-2-Z-(2-carboxy- prop-2-yloxyimino)acetamido]-acetoxymethyl-3-cephem-4-carboxylat

Eine Lösung von 2,3 g (3,35 mMol) 7ß-[2-(5-tert.-Butoxy- carbonylamino-1,2,4-thiadiazol-3-yl)-2-Z-(2-tert.-butoxycarbonyl- prop-2-yloxyimino)-acetamido]-3-acetoxymethyl-3-cephem-4-carbonsäure in 12,54 ml (16,38 mMol) Trifluoressigsäure und 2,10 ml (19,28 mMol) Anisol wird bei Zimmertemperatur 1 Stunde gerührt. Das Reaktionsgemisch wird bei 30° Badtemperatur am Vakuum zur Trockne eingedampft, der Rückstand mit Aether verrührt und das Produkt abfiltriert. Dann wird das Produkt in 28 ml Methanol gelöst und mit Natrium-äthylhexanoat versetzt. Man rührt die Lösung in 50 ml Essigsäureäthylester ein. Das ausgefallene Produkt wird abfiltriert und am Hochvakuum getrocknet. Man erhält die Titelverbindung mit dem Rf-Wert 0,78 (UPC12-Platten, Wasser/Acetonitril 4:1); IR-Spektrum (Nujol): charakteristische Absorptionsbanden bei 3.04; 5.68; 6.24; 6.57 µ.

### Beispiel 3:

### 7ß-[2-(5-Amino-1,2,4-thiadiazol-3-yl)-2-methylcarbamoyloxyimino- acetamido]-3-(4-carboxypyridiniomethyl)-3-cephem-4-carboxylat (A) und

### 7β-[2-(5-Amino-1,2,4-thiadiazol-3-yl)-2-hydroximino-acetamido]-3-(4-carboxypyridiniomethyl)-3-cephem-4-carboxylat (B)

Aus 1,50 g (2,88 mMol) Natrium-7β-[2-(5-amino-1,2,4-thiadiazol-3-yl)-2-methylcarbamoyloxyiminoacetamido]-3-acetoxymethyl-3-cephem-4-carboxylat, 0,49 g (3,98 mMol) Isonicotinsäure und 4,90 g (32,7 mMol) Natriumjodid in 4,2 ml Wasser erhält man analog Beispiel 1 die Titelverbindung A mit einem Rf-Wert von 0,30 (UPC₁₂-Platten, Wasser/Acetonitril 6:1) und die Titelverbindung B mit einem Rf-Wert von 0,55 (UPC₁₂-Platten, Wasser/Acetonitril 6:1).

Das Ausgangsmaterial kann wie folgt hergestellt werden:

### b) 2-(5-tert.-Butoxycarbonylamin.-1,2,4-thiadiazol-3-yl)-2-hydroxy- iminoessigsäure

Eine Lösung von 8,0 g (26,46 mMol) 2-(5-tert.-Butoxycarbonyl- amino-1,2,4-thiadiazol-3-yl)-2-hydroxyiminoessigsäuremethylester in 175 ml Aethanol wird mit 8,6 g Kaliumhydroxid und 70 ml Wasser versetzt und während 5 Stunden bei 40° gerührt.. Das Reaktionsgemisch wird im Vakuum eingeengt, der Rückstand in Wasser gelöst, die Lösung mit verdünnter Salzsäure auf pH 8,5 gestellt und mit Aethylacetat extrahiert. Die wässrige Phase wird abgetrennt, mit 2 N HC1 auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Der Rückstand wird aus Diäthyläther kristallisiert. Man erhält die einheitliche Titelverbindung vom Rf-Wert 0,60 (Silicagel, s-Butanol/Eisessig/Wasser 67:10:23); F. 158-160° (Zers.); IR-Spektrum (Nujol): charakteristische Absorptionsbanden bei 3.10; 5.85; 8.65 u.

### c) 2-(5-tert.-Butoxycarbonylamino-1,2,4-thiadiazol-3-yl)-2-Z-methyl- carbamoyliminoessigsäure

Eine Lösung von 2,85 g (8,95 mMol) 2-(5-tert.-Butoxycarbonyl- amino-1,2,4-thiadiazol-3-yl)-2-hydroxyiminoessigsäure in 20 ml Tetrahydrofuran wird bei 0-5° mit 10 ml Methylisocyanat versetzt und bei dieser Temperatur während 2 Stunden gerührt. Das Reaktionsgemisch wird mit n-Hexan versetzt und der entstandene Niederschlag abgenutscht. Nach Umkristalisation des Niederschlages aus Methylenchlorid erhält man die einheitliche Titelverbindung vom Rf-Wert 0,25 (UPC₁₂ -Platten, Wasser/Acetonitril 6:1); F. 124-127°; IR-Spektrum (Nujol): charakteristische Absorptionsbanden bei 5.75; 5.85; 6.40 µ.

### d) 7ß-[2-(5-tert.-Butorycarbonylamino-1,2,4-thiadiazol-3-yl)-2-Z-methylcarbamoyloxyiminoacetamido]-3-acatoxymethyl-3-cephem-4-carbonsäure-diphenylmethylester

1,05 g (3,70 mMol) 2-(5-tert.-Butoxycarbonylamino-1,2,4-thiadiazol-3-yl)-2-Z-methylcarbamoyloxyiminoessigsäure und 0,4 ml N-Methylmorpholin werden bei -5° zu einer gerührten Suspension eines Vilsmeier-Reagenses (hergestellt aus 0,031 ml Oxalylchlorid und 0,29 ml N,N-Dimethylformamid in 10 ml Aethylacetat) gegeben. Das Gemisch wird während 30 Minuten bei 0° gerührt und anschliessend auf -10° abgekühlt. Nach Zugabe von 1,357 g (3,1 mMol) 7ß-Amino-3-acetoxymethyl-3-cephem-4-carbonsäurediphenylmethylester wird während 2 1/2 Stunden bei 0° gerührt, dann mit Aethylacetat verdünnt und nacheinander mit Wasser, Phosphatpuffer pH 8,0 und wässriger Natriumchloridlösung gewaschen, über Magnesiumsulfat getrocknet und im Vakuum eingeengt. Ein Teil des Rohproduktes wird durch präparative Schichtchromatographie mit Aethylacetat gereinigt. Man erhält die einheitliche Titelverbindung vom Rf-Wert 0,60 (Silicagel, Aethylacetat); IR-Spetrum (CH₂Cl₂): charakteristische Absorptionsbanden bei 2.95; 3.10; 5.65; 5.80; 5.95; 8.20 µ.

### e) Natrium-7β-[2-(5-amino-1,2,4-thiadiazol-3-yl)-2-Z-mthylcarbamoyl- oayiminoacetamido]-3-acetoaymethyl-3-cephem-4-carboxylat

Aus 0,519 g (0,68 mMol) 7β-[2-(5-tert.-Butoxycarbonylamino-1,2,4-thiadiazol-3-yl)-2-Z-methylcarbamoyloxyiminoacetamido]-3-acetoxymethyl-3-cephem-4-carbonsäurediphenylmethylester in 1,1 ml Methylenchlorid, 0,37 ml Anisol und 5 ml Trifluoressigsäure erhält man analog Beispiel 2h die Titelverbindung vom Rf-Wert 0,40 (UPC₁₂-Platten, Wasser/Acetonitril 6:1); IR-Spektrum (Nujol): charakteristische Absorptionsbanden bei 3.0; 5.67; 6.22; 8.10 µ.

### Beispiel 4: 7ß-[2-(5-Amino-1,2,4-thiadiazol-3-yl)-2-Z-methoxyimino- acetamidol-3-(3-carboxymethyl-pyridiniomethyl)-3-cephem-4-carboxylat

Ausgehend von 0,77 g (1,48 mMol) Natrium-7β-[2-(5-amino-1,2,4-thiadiazol-3-yl)-2-Z-methoxyiminoacetamido]-3-acetoxymethyl-3-cephem-4-carboxylat, 2,63 g (1,93 mMol) 3-Pyridylessigsäure und 2,52 g Natriumjodid in 2,15 ml Wasser erhält man analog Beispiel 1 die Titelverbindung vom Rf-Wert 0,20 (UPC₁₂-Platten, Wasser/Acetonitril 6:1); IR-Spektrum (Nujol): charakteristische Absorptionsbanden bei 3.0; 5.67; 6.22 und 9.62 µ.

### Beispiel 5: 7β-[2-(5-Amino-1,2,4-thiadiazol-3-yl)-2-Z-2-carboxyprop-2-yloxyiminoacetamido]-3-(3-methyl-l-triazoliomethyl)-3-cephem-4-carboxylat

Ausgehend von 0,573 g (1 mMol) Dinatrium-7β-[2-(5-amino-1,2,4-thiadiazol-3-yl)-2-Z-(2-carboxyprop-2-yloxyimino)acetamido]-3-acetoxymethyl-3-cephem-4-carboxylat, 0,3 g (3,57 mMol) 1-Methyl-1,2,3-triazol und 1,9 g Natriumjodid in 0,3 ml Wasser erhält man analog Beispiel 2 die Titelverbindung.

### Beispiel 6: 7β-[2-(5-Amino-1,2,4-thiadiazol-3-yl)-2-Z-2-carboxyprop-2-yloxyiminoacetamido]-3-(2-methyl-1-pyrazoliomethyl)-3-cephem-4-carboxylat

Ausgehend von 0,286 g (0,5 mMol) Dinatrium-7ß-[2-(5-amino-1,2,4-thiadiazol-3-yl)-2-Z-2-carboxyprop-2-yloxyiminoacet amido]-3-acetoxymethyl-3-cephem-4-carboxylat, 0,4 g (4,8 mMol) N-Methylpyrazol und 1,0 g Natriumjodid in 0,3 ml Wasser erhält man analog Beispiel 2 die Titelverbindung vom Ri-Wert 0,39 (UPC₁₂-Platten, Wasser/Acetonitril 9:1).

### Beispiel 7: 7β-[2-(5-Amino-1,2,4-thiadiazol-3-yl)-2-Z-methoxyimino- acetamido]-3-(3-methyl-1-triazoliomethyl)-3-cephem-4-carboxylat

Ausgehend von 0,24 g (0,5 mMol) Natrium-7β-[2-(5-amino-1,2,4-thiadiazol-3-yl)-2-Z-methoxyimino-acetamido]-3-acetoxymethyl-3- _ cephem-4-carboxylat, 0,3 g 1-Methyl-1,2,3-triazol und 1,9 g Natriumjodid in 0,3 ml Wasser erhält man analog Beispiel 1 die Titelverbindung.

### Beispiel 8: 7β-[2-(5-Amino-1,2,4-thiadiazol-3-yl)-2-Z-methoxyimino- acetamido]-3-(2-methyl-1-pyrazoliomethyl)-3-cephem-4-carboxylat

Ausgehend von 0,478 g (1,0 mMol) Natrium-7β-[2-(5- amino-1,2,4-thiadiazol-3-yl)-2-Z-methoxyiminoacetamido]-3-acetoxymethyl-3-cephem-4-carboxylat, 0,115 g (1,4 mMol) N-Methylpyrazol und 7 g Natriumjodid in 1 ml Wasser erhält man analog Beispiel 1 die Titelverbindung mit einem Rf-Wert von 0,22 (UPC₁₂-Platten, Wasser/Acetonitril 6:1); IR-Spektrum (Nujol): charakteristische Absorptionsbanden bei 3.0; 5.75; 5.95; 6.20 und 9.62µ.

### Beispiel 9: 7β-[2-(5-Amino-1,2,4-thiadiazol-3-yl)-2-Z-methoxyimino- acetamido]-3-(3-brompyridiniomethyl)-3-cephem-4-carboxylat:

Ausgehend von 1,9 g (3,97 mMol) Natrium-7β-[2-(5-amino-1,2,4-thiadiazol-3-yl)-2-Z-methoxyiminoacetamido]-3-acetoxymethyl-3-cephem-4-carboxylat, 0,54 ml (5,6 mMol) 3-Brompyridin und 6,84 g Natriumjodid in 4 ml Wasser erhält man analog Beispiel 1 die Titelverbindung vom Rf-Wert 0,20 (UPC₁₂-Platten, Wasser/Acetonitril 6:1); IR-Spektrum (Nujol): charakteristische Absorptionsbanden bei 2.95; 5.75; 6.20; 6.70; 9.60 µ.

### Beispiel 10: 7β-[2-(5-Amino-1,2,4-thiadiazol-3-yl)-2-Z-methoxyimino- acetamido]-3-(4-brompyridiniomethyl)-3-cephem-4-carboxylat

Ausgehend von 1,44 g (3,0 mMol) Natrium-7β-[2-(5-amino-1,2,4-thiadiazol-3-yl)-2-Z-methoxyiminoacetamido]-3-acetoxymethyl-3-cephem-4-carboxylat, 0,5319 g (3,3 mMol) 4-Brompyridin, 5,2 g Natriumjodid und 3,15 ml Wasser erhält man analog Beispiel 1 die Titelverbindung vom R_{f} -Wert 0,22 (UPC₁₂-Platten, Wasser/Acetonitril 6:1); IR-Spektrum (Nujol): charakteristische Absorptionsbanden bei 2.95; 5.70; 6.17; 9.65 p.

### Beispiel 11: 7ß-[2-(5-Amino-1,2,4-thiadiazol-3-yl)-2-Z-methoxyimino- acetamido]-3-(4-hydroxymethylpyridiniomethyl)-3-cephem-4-carboxylat

2,87 g (6,0 mMol) Natrium-7β-[2-(5-amino-1,2,4-thiadiazol-3-yl)-2-methoxyiminoacetamido]-3-acetoxymethyl-3-cephem-4-carboxylat werden zu einer auf 70° vorgewärmten Lösung von 9,9 g Natriumjodid in 5,0 ml Wasser gegeben. Nach Zugabe von 0,72 g (6,6 mMol) 4-Hydroxymethylpyridin und 0,36 ml Essigsäure wird 5 Stunden bei 70° gerührt. Die Reaktionslösung wird auf Raumtemperatur abgekühlt und in 120 ml 0° kaltem Aethanol eingetragen. Der gebildete Niederschlag wird abfiltriert, mit Aethanol gewaschen und getrocknet. Nach chromatographischer Reinigung des Rohproduktes an XAD-2 Harz (Wasser/Isopropylalkohol 95:5) und nachfolgender Dickschichtchromatographie an Silicagel "Opti UPC₁₂" mit Wasser/Acetonitril 4:1 erhält man die einheitliche Titelverbindung vom Rf-Wert 0,45 (UPC₁₂-Platten, Wasser/Acetonitril 6:1); IR-Spektrum (Nujol): charakteristische Absorptionsbanden bei 2.95; 5.70; 6.20; 9.80 µ.

### Beispiel 12: 7β-[2-(5-Amino-1,2,4-thiadiazol-3-yl)-2-Z-methoxyimino- acetamido]-3-[4-(2-sulfoäthyl)-pyridiniomethyl]-3-cephem-4-carboxylat- natriumsalz

Ausgehend von 0,478 g (1,0 mMol) Natrium-7ß-[2-(5-amino-1,2,4-thiadiazol-3-yl)-2-Z-methoxyiminoacetamido]-3-acetoxymethyl-3-cephem-4-carboxylat, 0,294 g (1,4 mMol) Natrium-4-Pyridinäthansulfonat und 1,71 g Natriumjodid in 1 ml Wasser erhält man analog Beispiel 1 die Titelverbindung vom Rf-Wert 0,40 (UPC₁₂-Platten, Wasser/Acetonitril 95:5); IR-Spektrum (Nujol): charakteristische Absorptionsbanden bei 2.95; 5.65; 6.20; 9.80 µ.

### Beispiel 13: Natrium-7ß-[2-(5-amino-1,2,4-thiadiazol-3-yl)-2-Z-methoxy- iminoacetamido]-3-(2-amino-3-carboxypyridiniomethyl)-3-cephem-4-carboxylat

Ausgehend von 1,90 g (4,0 mMol) Natrium-7ß-[2-(5-amino-l,2,4-thiadiazol-3-yl)-2-Z-methoxyiminoacetamido]-3-acetoxymethyl-3-cephem-4-carboxylat, 0,83 g (6,0 mMol) 2-Aminonicotinsäure und 6,8 g Natriumjodid in 4 ml Wasser erhält man die Titelverbindung vom Rf-Wert 0,35 (UPC₁₂-Platten, Wasser/Acetonitril 95:5).

### Beispiel 14: 7β-[2-(5-Amino-1,2,4-thiadiazol-3-yl)-2-Z-methoxyimino- acetamido]-3-(2-amino-5-carbamoylpyridiniomethyl)-3-cephem-4-carboxylat

Ausgehend von 0,478 g (1,0 mMol) Natrium-7ß-[2-(5-amino-1,2,4-thiadiazol-3-yl)-2-Z-methoxyiminoacetamido]-3-acetoxymethyl-3-cephem-4-carboxylat, 0,192 g (1,4 mMol) 2-Amino-5-earbamoylpyridin und 1,71 g Natriumjodid in 1 ml Wasser erhält man analog Beispiel 1 die Titelverbindung vom Rf-Wert 0,28 (UPC₁₂-Platten, Wasser/Acetonitril 6:1); IR-Spektrum (Nujol):charakteristische Absorptionsbanden bei 3.10; 5.65; 7.40; 9.60 p.

### Beispiel 15: Natrium-7β-[2-(5-amino-1,2,4-thiadiazol-3-yl)-2-Z-methoxyiminoacetamido]-3-[3-(2-carboxyvinyl)-pyridiniomethyl]-3-cephem-4-carboxylat

Ausgehend von 0.478 (1,0 mMol) Natrium-7β-[2-(5-amino-1,2,4-thiadiazol-3-yl)-2-Z-methoxyiminoacetamido]-3-acetoxymethyl-3-cephem-4-carboxylat, 0,241 g (1,4 mMol) Natrium-3-(3-pyridyl)-acrylat und 1,71 g Natriumjodid in 1 ml Wasser erhält man analog Beispiel 1 die Titelverbindung vom Rf-Wert 0,85 (UPC₁₂-Platten, Wasser/Acetonitril 1:1); IR-Spektrum (Nujol): charakteristische Absorptionsbanden bei 2.95; 5.67; 6.15; 9.65; 10.20 µ.

### Beispiel 16: Natrium-7β-[2-(5-amino-1,2,4-thiadiazol-3-yl)-2-Z-methoxyiminoacetamido]-3-(3-sulfopyridiniomethyl)-3-cephem-4-carboxylat

Ausgehend von 1,9 g (4,0 mMol) Natrium-7ß-[2-(5-amino-1,2,4-thiadiazol-3-yl)-2-Z-methoxyiminoacetamido]-3-acetoxymethyl-3-cephem-4-carboxylat, 1,02 g (5,6 mMol) Natrium-pyridin-3-sulfonat und 6,84 g Natriumjodid in 3 ml Wasser erhält man analog Beispiel 1 die Titelverbindung-vom Rf-Wert 0,35 (UPC₁₂-Platten, Wasser/Acetonitril 6:1); IR-Spektrum (Nujol): charakteristische Absorptionsbanden bei 3.10; 5.82; 8.15; 9.10 µ.

### Beispiel 17: 7β-[2-(5-Amino-1,2,4-thiadiazol-3-yl)-2-Z-methoxyimino- acetamido]-3-(4-methoxymethylpyridiniomethyl)-3-cephem-4-carboxylat

Ausgehend von 0,478 g (1,0 mMol) Natrium-7ß-[2-(5-amino-1,2,4-thiadiazol-3-yl)-2-Z-methoxyiminoacetamido]-3-acetoxymethyl-3-cephem-4-carboxylat, 0,16 g (1,3 mMol) 4-Methoxymethylpyridin, und 1,70 g Natriumjodid in 1,0 ml Wasser erhält man analog Beispiel 1 die Titelverbindung vom Rf-Wert 0,30 (UPC₁₂-Piatten, Wasser/Acetonitril 6:1).

### Beispiel 18: 7β-[2-(5-Amino-1,2,4-thiadiazol-3-yl)-2-Z-methoxyimino- acetamido]-3-(4-thiocarbamoylpyridiniomethyl)-3-cephem-4-carboxylat.

Ausgehend von 0,478 g (1,0 mMol) Natrium-7β-[2-(5-amino-1,2,4-thiadiazol-3-yl)-2-Z-methoxyiminoacetamido]-3-acetoxymethyl-3-cephem-4-carboxylat, 0,166 g (1,20 mMol) Thioisonicotinsäureamid und 1,65 g Natriumjodid in 0,8 ml Wasser erhält man analog Beispiel 1 die Titelverbindung vom Rf-Wert 0,37 (UPC₁₂-Platten, Wasser/Acetonitril 4:1).

### Beispiel 19: 7β-[2-(5-Amino-1,2,4-thiadiazol-3-yl)-2-Z-methoxyimino- acetamido]-3-(3-cyanopyridiniomethyl)-3-cephem-4-carboxylat

Ausgehend von 0,956 g (2,0 mMol) Natrium-7ß-[2-(5-amino-1,2,4-thiadiazol-3-yl)-2-Z-methoxyiminoacetamido]-3-acetoxymethyl-3-cephem-4-carboxylat, 0,25 g (2,40 mMol) 3-Cyanpyridin und 3,4 g Natriumjodid in 3 ml Wasser erhält man analog Beispiel 1 die Titelverbindung vom Rf-Wert 0,30 (UPC₁₂-Platten, Wasser Acetonitril 6:1); IR-Spektrum (Nujol): charakteristische Absorptionsbanden bei 2.95; 4.65; 5.70; 6.20 µ.

### Beispiel 20: 7β-[2-(5-Amino-1,2,4-thiadiazol-3-yl)-2-Z-methoxyimino- acetamido]-3-(3-cyanomethylpyridiniomethyl)-3-cephem-4-carboxylat

Ausgehend von 0,478 g (1,0 mMol) Natrium-7ß-[2-(5-amino- l,2,4-thiadiazol-3-yl)-2-Z-methoxyiminoacetamido]-3-acetoxymethyl-3-cephem-4-carboxylat, 0,156 g (1,5 mMol) 3-Cyanomethylpyridin und 1,70 g Natriumjodid in 1,2 ml Wasser erhält man analog Beispiel 1 die Titelverbindung vom Rf-Wert 0,15 (UPC₁₂-Platten, Wasser/Acetonitril 9:1); IR-Spektrum (Nujol): charakteristische Absorptionsbanden bei 3.0; 4.65; 5.67 µ.

### Beispiel 21: 7β-[2-(5-Amino-1,2,4-thiadiazol-3-yl)-2-methoxyimino- acetamido]-3-(3-hydroxymethplpyridiniomethyl)-3-cephem-4-carboxylat

Ausgehend von 0,478 g (1,0 mMol) Natrium-7ß-[2-(5-amino-1,2,4-thiadiazol-3-yl)-2-Z-methoxyiminoacetamido]-3-acetoxymethyl-3-cephem-4-carboxylat, 0,164.g (1,5 mMol) 3-Hydroxymethylpyridin und 1,70 g Natriumjodid in 1 ml Wasser und 0,05 ml Essigsäure erhält man analog Beispiel 11 die Titelverbindung vom Rf-Wert 0,40 (UPC₁₂ -Platten, Wasser/Acetonitril 9:1); IR-Spektrum (Nujol): charakteristische Absorptionsbanden bei 2.95; 5.67; 6.20 µ.

### Beispiel 22: 7β-[2-(5-Amino-1,2,4-thiadiazol-3-yl)-2-methylcarbamoyl- oxyiminoacetamido-3-(4-hydroxymethylpyridiniomethyl)-3-cephem-4-carboxylat (A) und

### 7β-[2-(5-Amino-1,2,4-thiadiazol-3-yl)-2 hydroxyiminoacetamido]-3-(4-hydroxymethylpyridiniomethyl)-3-cephem-4-carboxylat (B)

Aus 1,042 g (2,0 mMol) Natrium 7β-[2-(5-amino-1,2,4-thiadiazol-3-yl)-2-methylcarbamoyloxyiminoacetamido]-3-acetoxymethyl-3-cephem-4-carboxylat, 0,24 g (2,2 mMol) 4-Hydroxymethylpyridin und 3,5 g Natriumjodid in 2,1 ml Wasser erhält man analog Beispiel 1 die Titelverbindung A vom Rf-Wert 0,40 (UPC₁₂-Platten, Wasser/Acetonitril 6:1) und die Titelverbindung B vom Rf-Wert 0,60 (UPC₁₂-Platten, Wasser/ Acetonitril 6:1).

### Beispiel 23: Natrium-7β-[2-(5-amino-1,2,4-thiadiazol-3-yl)-2-Z-methoxyiminoacetamido]-3-(3-carboxypyridiniomethyl)-3-cephem-4-carboxylat

Ausgehend von 0,478 g (1,0 mMol) Natrium-7β-[2-(5-amino-1,2,4-thiadiazol-3-yl)-2-Z-methoxyimino-acetamido]-3-acetoxymethyl-3-cephem-4-carboxylat, 0,20 g (1,64 mMol) Nicotinsäure und 1,70 g Natriumjodid in 1 ml Wasser erhält man analog Beispiel 1 die Titelverbindung vom Rf-Wert 0,20 (UPC₁₂-Platten, Wasser/Acetonitril 9:1); IR-Spektrum (Nujöl): charakteristische Absorptionsbanden bei 3.0; 5.70; 6.20 µ.

### Beispiel 24: 7β-[2-(5-Amino-1,2,4-thiadiazol-3-yl)-2-Z-methoxyimino- acetamido]-3-(2-carboxymethyl-l-pyrazoliomethyl)-3-cephem-4-carbonsäure (A) und

### 7β-(2-(5-Amino-1,2,4-thiadiazol-3-yl)-2-Z-methoxyiminoacetamido]-3-(1-carboxymethyl-1-pyrazoliomethyl)-3-cephem-4-carbonsäure (B)

Aus 1,44 g (3,0 mMol) Natium-7ß-[2-(5-amino-l,2,4-thiadiazol-3-yl)-2-methoxyiminoacetamido]-3-acetoxymethyl-3-cephem-4-carboxylat, 0,53 g (4,2 mMol) Pyrazolylessigsäure und 5,15 g Natriumjodid in 3 ml Wasser erhält man analog Beispiel 1 ein Gemisch der Titelverbindung A mit dem Rf-Wert 0,75 (UPC₁₂-Platten, Wasser/Acetonitril 6:1) und B mit dem Rf-Wert 0,70 (UPC₁₂-Platten, Wasser/Acetonitril 6:1).

### Beispiel 25: Dinatrium-7β-[2-(5-amino-1,2,4-thiadiazol-3-yl)-2-Z-2-carboxyprop-2-yloxyiminoacetamido]-3-(2-carboxymethyl-l-pyrazolio- methyl)-ceph-3-em-4-carboxylat

Zu einer Lösung von 0,9 g (7,14 mMol) Pyrazolylessigsäure und 0,9 g Natriumjodid in 2,0 ml Wasser wird bei 70° eine Lösung von 2,8 g (6,0 mMol) Natrium-7β-[2-(5-amino-1,2,4-thiadiazol-3-yl)-2-methoxyiminoacetamido]-3-acetoxymethyl-3-cephem-4-carboxylat in 4 ml Wasser gegeben. Man führt die Reaktion analog Beispiel 1 durch und erhält die Titelverbindung; IR-Spektrum (Nujol) 2.90; 5.65; 6.22 und 7.05 µ.

### Beispiel 26: 7β-[2-(5-Amino-1,2,4-thiadiazol-3-yl)-2-Z-methoxyimino- acetamido]-3-(2-äthoxycarbonylmethyl-1-pyrazoliomethyl)-3-cephem-4-carboxylat

Aus 1,0 g (2,1 mMol) Natrium-7ß-[2-(5-amino-l,2,4-thiadiazol-3-yl)-2-methoxyiminoacetamido]-3-acetoxymethyl-3-cephem-4-carboxylat, 3,08 g (20 mMol) Pyrazolylessigsäureäthylester und 3,73 g (25 mMol) Natriumjodid erhält man analog Beispiel 1 die Titelverbindung; IR-Spektrum (Nujol): 5.66; 5.77; 6.23µ.

### Beispiel 27: In analoger Weise kann man unter Verwendung der geeigneten Ausgangsstoffe die folgenden Verbindungen erhalten:

7β-[2-(5-Amino-1,2,4-thiadiazol-3-yl)-2-Z-hydroxyiminoacetamido]-3-(2-methyl-l-pyrazoliomethyl)-3-cephem-4-carboxylat; Rf: 0.40 (Wasser/ Acetonitril 6:1); IR: 3.0; 5.67; 6.20; 8.30 µ;
7β-[2-(5-Amino-1,2,4-thiadiazol-3-yl)-2-Z-hydroxyimino-acetamido]-3-(3-methyl-l-triazoliomethyl)-3-cephem-4-carboxylat; Rf: 0,25 (Wasser/ Acetonitril 6:1); IR: 5.65; 6,20 µ;
7β-[2-(5-Amino-1,2,4-thiadiazol-3-yl)-2-Z-(2-carboxyprop-2-yloxyimino)-acetamido]-3-(4-hydroxymethylpyridiniomethyl)-3-cephem-4-carboxylat; Rf: 0.50 (Wasser/Acetonitril 6:1); IR: 5.67; 5.95; 9.20 µ;
7ß-[2-(5-Amino-1,2,4-thiadiazol-3-yl)-2-Z-hydroxyimino-acetamido]-3-(4-hydroxymethylpyridiniomethyl)-3-cephem-4-earboxylat;
7β-[2-(5-Amino-1,2,4-thiadiazol-3-yl)-2-Z-hydroxyimino-acetamido]-3-(3-sulfopyridiniomethyl)-3-cephem-4-carboxylat; Rf: 0.45 (Wasser/ Acetonitril 6:1); IR: 3.0; 5.75; 9.15 µ;
7β-[2-(5-Amino-1,2,4-thiadiazol-3-yl)-2-Z-(2-carboxyprop-2-yloxyimino)-acetamido]-3-(3-sulfopyridiniomethyl)-3-cephem-4-carboxylat; Rf: 0.50 (Wasser/Acetonitril 6:1); IR: 3.05; 5.67; 5.95 µ.
7β-[2-(5-Amino-1,2,4-thiadiazol-3-yl)-2-Z-hydroxyimino-acetamido]-3-(3-carboxymethyl-4-carbamoylpyridiniomethyl)-3-cephem-4-carboxylat; Rf: 0.35 (Wasser/Acetonitril 4:1); IR: 3.0; 5.67; 6.20 p;
7β-[2-(5-Amino-1,2,4-thiadiazol-3-yl)-2-Z-methoxyimino-acetamido]-3-(3-carboxymethyl-4-carbamoylpyridiniomethyl)-3-cephem-4-carboxylat; Rf: 0.30 (Wasser/Acetonitril 4:1); IR: 3.0; 5.65; 6.20 µ;
7β-[2-(5-Amino-1,2,4-thiadiazol-3-yl)-2-Z-(2-carboxyprop-2-yloxyimino)-acetamido]-3-(3-carboxymethyl-4-carbamoylpyridiniomethyl)-3-cephem-4-carboxylat; Rf: 0.37 (Wasser/Acetonitril 4:1); IR: 3.0; 5.67; 6.20 p;
7β-[2-(5-Amino-1,2,4-thiadiazol-3-yl)-2-Z-hydroxyimino-acetamido]-3-(3-carboxy-4-carbamoylpyridiniomethyl)-3-cephem-4-carboxylat; Rf: 0.45 (Wasser/Acetonitril 6:1); IR: 3.0; 5.65; 6.17 µ;
7ß-[2-(5-Amino-1,2,4-thiadiazol-3-yl)-2-Z-methoxyiminoacetamido]-3-(3-carboxy-4-carbamoylpyridiniomethyl)-3-cephem-4-carböxylat; Rf: 0.35 (Wasser/Acetonitril 6:1); IR: 5.67; 6.20 µ;
7β-[2-(5-Amino-1,2,4-thiadiazol-3-yl)-2-Z-(2-carboxyprop-2-yloxyimino)-acetamido]-3-(3-carboxy-4-carbamoylpyridiniomethyl)-3-cephem-4-carboxylat; Rf: 0.35 (Wasser/Acetonitril 6:1); IR: 5.67; 5.95 µ;
7β-[2-(5-Amino-1,2,4-thiadiazol-3-yl)-2-Z-hydroxyiminoacetamido]-3-(3-brompyridiniomethyl)-3-cephem-4-carboxylat; Rf: 0.35 (Wasser/Acetonitril 6:1); Rf: 0.35 (Wasser/Acetonitril 6:1); IR: 3.0; 5.65; 6.20 µ;
7β-[2-(5-Amino-1,2,4-thiadiazol-3-yl)-2-Z-(2-carboxyprop-2-yloxyimino)-acetamido]-3-(3-brompyridiniomethyl)-3-cephem-4-carboxylat; Rf: 0,20 (Wasser/Acetonitril 9:1); IR: 3.0; 5.67 µ;
7β-[2-(5-Amino-1,2,4-thiadiazol-3-yl)-2-Z-hydroxyiminoacetamido]-3-(4-brompyridiniomethyl)-3-cephem-4-carboxylat; Rf: 0,30 (Wasser/Acetonitril 9:1); IR: 2.95; 5.65; 6.17 µ;
7β-[2-(5-Amino-1,2,4-thiadiazol-3-yl)-2-Z-(2-carboxyprop-2-yloxyimino)-acetamido]-3-(4-brompyridiniomethyl)-3-cephem-4-carboxylat: Rf: 0.20 (Wasser/Acetonitril 9:1); IR: 3.0; 5.70µ;
7ß-[2-(5-Amino-1.,2,4-thiadiazol-3-yl)-2-Z-methoxyiminoacetamido]-3-(4-carboxymethylthiopyridiniomethyl)-3-cephem-4-carboxylat IR: 2.95; 5.65; und
7ß-[2-(5-Amino-1,2,4-thiadiazol-3-yl)-2-Z-methoxyiminoacetamido]-3-(3-cyano-4-trifluoromethylpyridiniomethyl)-3-cephem-4-carboxylat; Rf: 0.40 (Wasser/Acetonitril 4:1); IR: 5.67; 6.20µ (Rf-Werte auf UPC12-Platten und IR-Spektren in Nujol).

### Beispiel 28: Trockenampullen oder Vials, enthaltend 0,5 g Wirksubstanz, z.B. Natriumsalz der 7β-[2-(5-Amino-1,2,4-thiadiazol-3-yl)-2-Z-methoxyimino-acetylamidoy-3-(4-carboxypyridiniomethyl)-3-cephem-4-carboxylat, können z.B. wie folgt hergestellt werden:

Zusammensetzung (für 1 Ampulle oder Vial)
Wirksubstanz 0,5 g
Mannit 0,05 g

Eine sterile wässrige Lösung der Wirksubstanz und des Mannits wird unter aseptischen Bedingungen in 5 ml-Ampullen oder 5 ml-Vials eingefüllt, und diese verschlossen und geprüft.

## Patentansprüche

Patentansprüche (für alle benannten Länder ausser Oesterreich): 1. Verbindungen der Formel 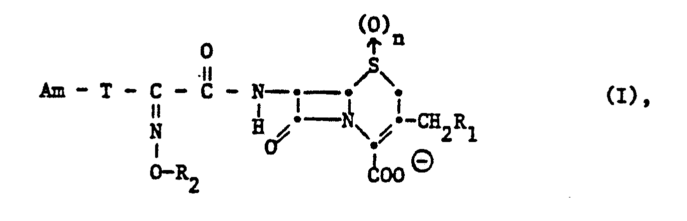 worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)- verbunden ist, R₁ eine unsubstituierte oder substituierte Pyrazoliogruppe, eine unsubstituierte oder substituierte Triazoliogruppe, eine Triniederalkylammoniogruppe oder eine Pyridiniogruppe der Formel 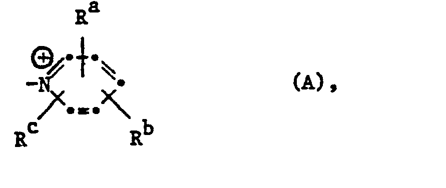 worin R^{a} durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl Niederalkanoyloxy, Niederalkanoyl, Carboxyl, Sulfo, Cyano oder Nitro, oder Hydroxysulfoniederalkyl bedeutet, R^{b} Carbamoyl oder Wasserstoff bedeutet oder die Bedeutungen von R^{a} hat und R^{c} Wasserstoff bedeutet oder die Bedeutungen von R^{a} hat, und R₂ Wasserstoff, unsubstituiertes oder substituiertes Niederalkyl oder Cycloalkyl oder unsubstituiertes oder substituiertes Carbamoyl bedeuten, und Salze von solchen Verbindungen, die eine salzbildende Gruppe aufweisen.

2. Verbindungen der Formel I, worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)- verbunden ist, R₁ eine 2-Niederalkyl-l-pyrazoliogruppe, eine 3-Niederalkyl-l-triazoliogruppe, eine Triniederalkylammoniogruppe oder eine Pyridiniogruppe der Formel A, worin R^{a} durch Cycloalkyl, Phenyl, Hydroxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl bedeutet, R^{b} Carbamoyl oder Wasserstoff bedeutet oder die Bedeutungen von R^{a} hat und R Wasserstoff bedeutet oder die Bedeutungen von R^{a} hat, und R₂ Wasserstoff, unsubstituiertes oder substituiertes Niederalkyl oder Cycloalkyl oder unsubstituiertes oder substituiertes Carbamoyl bedeuten, und Salze von solchen Verbindungen, die eine salzbildende Gruppe aufweisen.

3. Verbindungen der Formel I, worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)- verbunden ist, R₁ eine 2-Niederalkyl-1-pyrazoliogruppe, eine 3-Niederalkyl-l-triazoliogruppe, eine Triniederalkylammoniogruppe oder eine Pyridiniogruppe der Formel A, worin R^{a} durch Cycloalkyl, Phenyl, Hydroxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl bedeutet, R Carbamoyl oder Wasserstoff _{be}- deutet oder die Bedeutungen von R^{a} hat und R^{c} Wasserstoff bedeutet oder die Bedeutungen von R^{a} hat, und R₂ Wasserstoff, unsubstituiertes oder substituiertes Niederalkyl oder Cycloalkyl oder unsubstituiertes oder substituiertes Carbamoyl bedeuten, und Salze von solchen Verbindungen, die eine salzbildende Gruppe aufweisen.

4. Verbindungen der Formel I, gemäss Anspruch 1, worin n für 0 steht, Am Amino, Niederalkylamino, Diniederalkylamino oder geschütztes Amino, T einen Thiadiazolrest, welcher an einem Kohlenstoffatom mit Am und am anderen Kohlenstoffatom mit der Gruppe der Formel -C(=N-O-R₂)- verbunden ist, R₁ 2-Niederalkyl-l-pyrazolio, 2-Carboxyniederalkyl-l-pyrazolio, 1-Carboxyniederalkyl-l-pyrazolio, 2-Niederalkoxycarbonylniederalkyl-l-pyrazolio, 3-Niederalkyl₋l-triazolio oder eine Pyridiniogruppe der Formel A, worin R^{a} durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro oder Hydroxysulfoniederalkyl bedeutet, R Carbamoyl oder Wasserstoff bedeutet, und R₂ Wasserstoff, unsubstituiertes oder substituiertes Niederalkyl- oder Cycloalkyl oder unsubstituiertes oder substituiertes Carbamoyl bedeuten, wobei die Gruppe der Formel =N-O-R₂ insbesondere die syn(oder Z)-Form aufweist, sowie Salze, insbesondere pharmazeutisch verwendbare Salze von solchen Verbindungen der Formel I, die salzbildende Gruppen aufweisen.

5. Verbindungen der Formel I, gemäss Anspruch 3, worin n für 0 steht, Am Amino, Niederalkylamino, Diniederalkylamino oder geschütztes Amino, T einen Thiadiazolrest, welcher an einem Kohlenstoffatom mit Am und am anderen Kohlenstoffatom mit der Gruppe der Formel -C(-N-O-R₂)- verbunden ist, R₁ 2-Niederalkyl-l-pyrazolio, 3-Niederalkyl-1-triazolio oder eine Pyridiniogruppe der Formel A, worin R durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederälkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro oder Hydroxysulfoniederalkyl bedeutet, R Carbamoyl oder Wasserstoff bedeutet oder die Bedeutungen von R^{a} hat und R^{c} Wasserstoff bedeutet, und R2Wasserstoff, unsubstituiertes oder substituiertes Niederalkyl- oder Cycloalkyl oder unsubstituiertes oder substituiertes Carbamoyl bedeuten, wobei die Gruppe der Formel -N-O-R₂ insbesondere die syn(oder Z)-Form aufweist, sowie Salze, insbesondere pharmazeutisch verwendbare Salze von solchen Verbindungen der Formel I, die salzbildende Gruppen aufweisen.

6.. Verbindungen der Formel I, gemäss Anspruch 4, worin n für 0 steht, Am Amino, Niederalkylamino, z.B. Methylamino, oder geschütztes Amino, T einen Thiadiazolrest, welcher an einem Kohlenstoffatom mit Am und am anderen Kohlenstoffatom mit der Gruppe der Formel -C(=N-0-R₂)-verbunden ist, R₁ 2-Niederalkyl-l-pyrazolio, z.B. 2-Methyl-l-pyrazolio, 2-Carboxyniederalkyl-1-pyrazolio, z.B. 2-Carboxymethyl-l-pyrazolio, 1-Carboxyniederalkyl-l-pyrazolio, z.B. 1-Carboxymethyl-l-pyrazolio, 2-Niederalkoxycarbonylniederalkyl-l-pyrazolio, z.B. 2-Methoxycarbonylmethyl-l-pyrazolio, 3-Niederalkyl-l-triazolio, z.B. 3-Methyl-l-triazolio oder eine Pyridiniogruppe der Formel A, beispielsweise Hydroxyniederalkylpyridinio, z.B. 3- oder 4-Hydroxymethylpyridinio, Niederalkoxyniederalkylpyridinio, z.B. 4-Methoxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carbox^{y}vinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-car- bamoylpyridinio, Cyanohalogenmethylpyridinio, z.B. 3-Cyano-4-trifluor- methylpyridinio oder Aminocarboxypyridinio, z.B. 2-Amino-3-carboxypyridinio,und R₂ Wasserstoff, Niederalkyl, z.B. Methyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, bedeuten, wobei die Gruppe der Formel =N-O-R₂ die syn(oder Z)-Form aufweist, sowie Salze, insbesondere pharmazeutisch verwendbare Salze von solchen Verbindungen der Formel I, die salzbildende Gruppen aufweisen.

7. Verbindungen der Formel I gemäss Anspruch 5, worin n für 0 steht, Am Amino, Niederalkylamino, z.B. Methylamino, oder geschütztes Amino, T einen Thiadiazolrest, welcher an einem Kohlenstoffatom mit Am und am anderen Kohlenstoffatom mit der Gruppe der Formel -C(=N-O-R₂)-verbunden ist, R₁ 2-Niederalkyl-l-pyrazolio, z.B. 2-Methyl-l-pyrazolio, 3-Niederalkyltriazolio, z.B. 3-Methyl-l-triazolio oder eine Pyridiniogruppe der Formel A, beispielsweise Hydroxyniederalkylpyridinio, z.B. 3- oder 4-Hydroxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carba- moylpyridinio, Aminocarbamoylpyridinio, z.B. 2-Amino-5-carbamoyl- pyridinio, Carboxycarbamoylpyridinio, z.B. 3-Carboxy-4-carbamoyl- pyridinio, Cyanohalogenmethylpyridinio, z.B. 3-Cyano-4-trifluor- methylpyridinio oder Aminocarboxypyridinio, z.B. 2-Amino-3-carboxypyridinio, und R₂ Wasserstoff, Niederalkyl, z.B. Methyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, bedeuten, wobei die Gruppe der_Formel =N-0-R₂ die syn(oder Z)-Form aufweist, sowie Salze, insbesondere pharmazeutisch verwendbare Salze von solchen Verbindungen der Formel I, die salzbildende Gruppen aufweisen.

8. Verbindungen der Formel I, gemäss Anspruch 6, worin n für 0 steht, Am Amino, T einen 1,2,4-Thiadiazolrest, welcher in 5-Stellung mit Am und in 3-Stellung mit der Gruppe der Formel -C(=N-O-R₂)- verbunden ist, R₁ 2-Niederalkyl-l-pyrazolio, z.B. 2-Methyl-l-pyrazolio, 2-Carboxyniederalkyl-l-pyrazolio, z.B.. 2-Carboxymethyl-l-pyrazolio, 2-Niederalkoxycarbonylniederalkyl-l-pyrazolio, z.B. 2-Methoxycarbonylmethyl-l-pyrazolio, 3-Niederalkyl-1-triazolio, z.B. 3-Methyl-l-triazolio, oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Sulfoniederalkylpyridinio, z.B. 4-(2-Sulfoäthylpyridinio), Halogenpyridinio, z.B. 3-Brompyridinio, Carboxypyridinio, z.B. 4-Carboxypyridinio, Sulfopyridinio, z.B. 3-Sulfopyridinio oder Aminocarbamoylpyridinio, z.B. 2-Amino-5-carbamoylpyridinio, und R₂ Wasserstoff, Niederalkyl, z.B. Methyl, Carboxyniederalkyl, z.B.. 2-Carboxy-2-propyl, Carbamoyl oder Niederalkylcarbamoyl z.B. Methylcarbamoyl, bedeuten, wobei die Gruppe der Formel =N-O-R₂ die syn(oder Z)-Form aufweist, sowie Salze, in erster Linie pharmazeutisch verwendbare Salze von Verbindungen der Formel I.

9. Verbindungen der Formel I, gemäss Anspruch 7, worin n für 0 steht, Am Amino, T einen 1,2,4-Thiadiazolrest, welcher in 5-Stellung mit Am und in 3-Stellung mit der Gruppe der Formel -C(=N-O-R₂)- verbunden ist, R₁ 2-Niederalkyl-1-pyrazolio, z.B. 2-Methyl-l-pyrazolio, 3-Niederalkyl-1-triazolio, z.B. 3-Methyl-l-triazolio, oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Sulfoniederalkylpyridinio, z.B. 4-(2-Sulfo- äthylpyridinio), Halogenpyridinio, z.B. 3-Brompyridinio, Carboxypyridinio, z.B. 4-Carboxypyridinio, Sulfopyridinio, z.B. 3-Sulfopyridinio oder Aminocarbamoylpyridinio, z.B. 2-Amino-5-carbamoyl- pyridinio, und R₂ Wasserstoff, Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel =N-O-R₂- die syn(oder Z)-Form aufweist, sowie Salze, in erster Linie pharmazeutisch verwendbare Salze von Verbindungen der Formel I.

10. Verbindungen der Formel I, gemäss Anspruch 9, worin n für 0 steht, Am Amino, T einen 1,2,4-Thiadiazolrest, welcher in 5-Stellung mit Am und in 3-Stellung mit der Gruppe der Formel -C(=N-O-R₂)- verbunden ist, R₁ 2-Niederalkyl-l-pyrazolio, z.B. 2-Methyl-l-pyrazolio oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Halogenpyridinio, z.B. 3-Brompyridinio, oder Carboxypyridinio, z.B. 4-Carboxypyridinio, und R₂ Niederalkyl, z.B. Methyl oder Carboxyniederalkyl, z.B.. 2-Carboxy-2-propyl oder Aminocarbamoylpyridinio, z.B. 2-Amino-5-carbamoylpyridinio bedeuten, wobei die Gruppe der Formel =N-O-R₂- die syn(oder Z)-Form aufweist, sowie Salze, in erste Linie pharmazeutisch verwendbare Salze von Verbindungen der Formel I.

11. 7ß-[2-(5-Amino-1.,2,4-thiadiazol-3-yl)-2-Z-methoxyiminoacetamido]-3-(4-carboxypyridiniomethyl)-3-cephem-4-carboxylat, gemäss Anspruch 10.

12. 7β-[2-(5-Amino-1,2,4-thiadiazol-3-yl)-2-Z-2-carboxyprop-2-yloxy- iminoacetamido]-3-(3-carboxymethylpyridiniomethyl)-3-cephem-4-carboxylat, gemäss Anspruch 10.

13. 7ß-[2-(5-Amino-1,2,4-thiadiazol-3-yl)-2-Z-methoxyiminoacetamido]-3-(2-methyl-l-pyrazoliomethyl)-3-cephem-4-carboxylat, gemäss Anspruch 10.

14. 7ß-[2-(5-Amino-1,2,4-thiadiazol-3-yl)-2-Z-methoxyiminoacetamido]-3-[4-(2-sulfoäthyl)-pyridiniomethyl]-3-cephem-4-carboxylat, gemäss Anspruch 10.

15. 7ß-[2-(5-Amino-1,2,4-thiadiazol-3-yl)-2-Z-methoxyiminoacetamido]-3-(2-amino-5-carbamoylpyridiniomethyl)-3-cephem-4-carboxylat, gemäss Anspruch 10.

16.. 7β-[2-(5-Amino-1,2,4-thiadiazol-3-yl)-2-Z-methoxyiminoacet- amido]-3-(3-brompyridiniomethyl)-3-cephem-4-carboxylat, gemäss Anspruch 10.

17. 7ß-[2-(5-Amino-l,2,4-thiadiazol-3-yl)-2-Z-methoxyimino- acetamido]-3-(4-hydroxymethylpyridiniomethyl)-3-cephem-4-carboxylat, gemäss Anspruch 10.

18. Pharmazeutische Präparate enthaltend Verbindungen der Formel I gemäss Anspruch l, Hydrate oder pharmazeutisch verwendbare Salze von solchen Verbindungen.

19. Pharmazeutische Präparate enthaltend Verbindungen der Formel I gemäss Anspruch 3, Hydrate oder pharmazeutisch verwendbare Salze von solchen Verbindungen.

20. Verbindungen der Formel I gemäss Anspruch 1 zur Anwendung bei der Behandlung des menschlichen oder tierischen Körpers von bakteriellen Infektionen.

21. Verbindungen der Formel I gemäss Anspruch 3 zur Anwendung bei der Behandlung des menschlichen oder tierischen Körpers von bakteriellen Infektionen.

22. Verfahren zur Herstellung von Verbindungen der Formel I und Salzen von Verbindungen der Formel I, die eine salzbildende Gruppe aufweisen, dadurch gekennzeichnet, dass man
a) in einer Verbindung der Formel 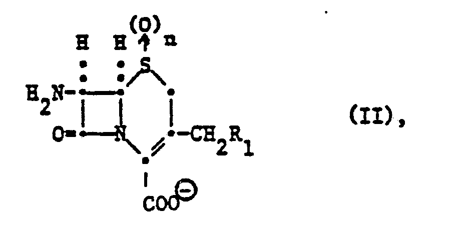 worin der Index n für 0 oder 1 steht, R₁ die unter Formel I genannten Bedeutungen hat, die 7ß-Aminogruppe gegebenenfalls durch eine die Acylierungsreaktion erlaubende Gruppe geschützt ist und die 4-Carboxygruppe gegebenenfalls geschützt ist, die 7ß-Aminogruppe durch Umsetzung mit einem den Acylrest einer Carbonsäure der Formel 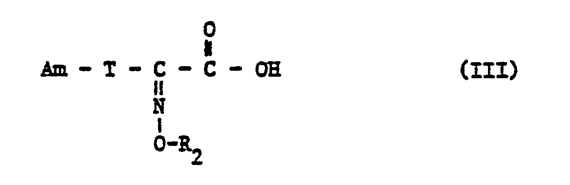 einführenden Acylierungsmittel, worin Am, T und R₂ die unter Formel 1 genannten Bedeutungen haben und vorhandene funktionelle Gruppen gegebenenfalls geschützt sind, acyliert oder
b) zur Herstellung einer Verbindung der Formel I, worin n für 0 steht, eine 2-Cephemverbindung der Formel 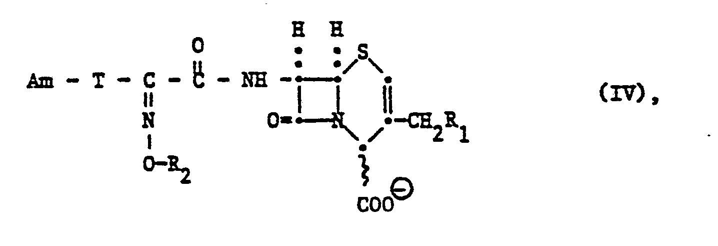 worin Am, T, R₁ und R₂ die unter Formel I genannten Bedeutungen haben und worin die 4-Carboxylgruppe sowie weitere funktionelle Gruppen gegebenenfalls geschützt sind, zur entsprechenden 3-Cephemverbindung isomerisiert oder
c) eine Verbindung der Formel 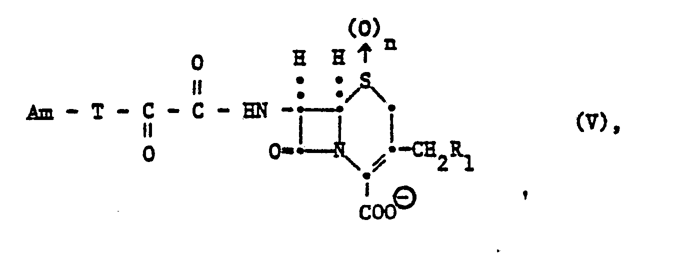 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Hydroxylaminderivat der Formel H₂N-O-R₂ (VI), worin R₂ die unter Formel I genannten Bedeutungen hat und funktionelle Gruppen gegebenenfalls geschützt sind, umsetzt oder
d) zur Herstellung einer Verbindung der Formel I, worin R₂ Wasserstoff bedeutet, eine Verbindung der Formel 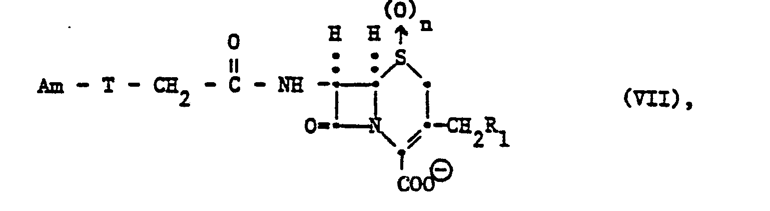 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Nitrosierungsmittel behandelt oder
e) zur Herstellung einer Verbindung der Formel I, worin Am eine freie oder eine sekundäre Aminogruppe, T einen 1,2,4-Thiadiazolrest darstellt, der in 5-Stellung mit Am und in 3-Stellung mit der Gruppe der Formel -C(=N-O-R₂)- verbunden ist, eine Verbindung der Formel 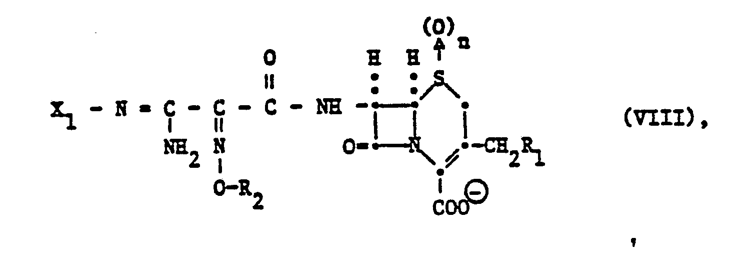 worin n für 0 oder 1 steht, X₁ Wasserstoff, Halogen oder Hydroxy bedeutet und R₁ die unter Formel I genannten Bedeutungen hat und worin die 4-Carboxylgruppe, sowie weitere funktionelle Gruppen gegebenenfalls geschützt sind, mit einem Salz der Rhodanwasserstoffsäure, bzw. mit einem Isorohodanwasserstoffsäureester oder, falls X₁ Wasserstoff bedeutet, mit Dirhodan behandelt oder
f) eine Verbindung der Formel 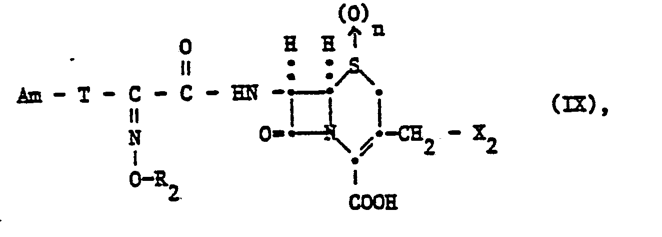 worin n für 0 oder 1 steht, Am, T und R₂ die unter Formel I genannten Bedeutungen haben und X₂ einen durch nucleophile Substitution ersetzbaren Rest darstellt und worin die 4-Carboxylgruppe sowie weitere funktionelle Gruppen gegebenenfalls geschützt sind, mit einer den Rest R₁ entsprechenden organischen tertiären Base umsetzt oder
g) zur Herstellung von Verbindungen der Formel I, worin Am eine primäre, sekundäre oder tertiäre Aminogruppe ist, eine Verbindung der Formel 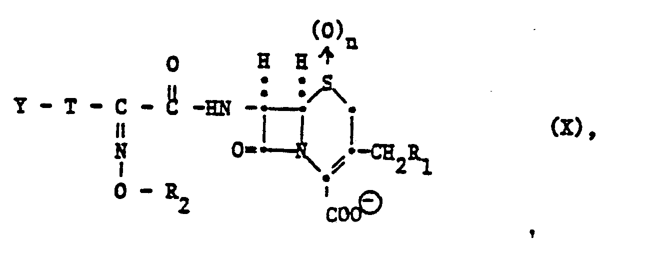 worin n für 0 oder 1 steht, T, R₁ und R₂ die unter Formel I genannten Bedeutungen haben und Y Halogen bedeutet, mit Ammoniak oder einem primären oder sekundären Amin Am-H oder einem Metallamid davon umsetzt und, wenn erwünscht, eine erhältliche Verbindung der Formel I in eine andere Verbindung der Formel I umwandelt und/oder, wenn erwünscht, eine erhältliche Verbindung der Formel I, worin n 0 bedeutet, in eine Verbindung der Formel I überführt, worin n 1 bedeutet, und/oder eine Verbindung der Formel I; worin n 1 bedeutet, in eine Verbindung der Formel I überführt, worin n 0 bedeutet, und/oder in einer erhältlichen Verbindung der Formel I in geschützter Form vorliegende funktionelle Gruppen in die freien funktionellen Gruppen überführt und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomeren auftrennt.

23. Die nach dem Verfahren gemäss Anspruch 22 herstellbaren Verbindungen.

24. Verfahren zur Herstellung von Verbindungen der Formel I gemäss Anspruch 3 und Salzen von solchen Verbindungen, die eine salzbildende Gruppe aufweisen, dadurch gekennzeichnet, dass man
a) in einer Verbindung der Formel III, worin der Index n für 0 oder 1 steht, R₁ die unter Formel I genannten Bedeutungen hat, die 7ß-Aminogruppe gegebenenfalls durch eine die Acylierungsreaktion erlaubende Gruppe geschützt ist und die 4-Carboxygruppe gegebenenfalls geschützt ist, die 7ß-Aminogruppe durch Umsetzung mit einem den Acylrest einer Carbonsäure der Formel III, einführenden Acylierungsmittel, worin Am, T und R₂ die unter Formel I genannten Bedeutungen haben und vorhandene funktionelle Gruppen gegebenenfalls geschützt sind, acyliert oder
b) zur Herstellung einer Verbindung der Formel I, worin n für 0 steht, eine 2-Cephemverbindung der Formel IV, worin Am, T, R₁ und R₂ die unter Formel I genannten Bedeutungen haben und worin die 4-Carboxylgruppe sowie weitere funktionelle Gruppen gegebenenfalls geschützt sind, zur entsprechenden 3-Cephemverbindung isomerisiert oder
c) eine Verbindung der Formel V, worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Hydroxylaminderivat der Formel H₂N-O-R₂ (VI), worin R₂ die unter Formel I genannten Bedeutungen hat und funktionelle Gruppen gegebenenfalls geschützt sind, umsetzt oder
d) zur Herstellung einer Verbindung der Formel I, worin R₂ Wasserstoff bedeutet, eine Verbindung der Formel VII, worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Nitrosierungsmittel behandelt, oder
e) zur Herstellung einer Verbindung der Formel I, worin Am eine freie oder eine sekundäre Aminogruppe, T einen 1,2,4-Thiadiazolrest darstellt, der in 5-Stellung mit Am und in 3-Stellung mit der Gruppe der Formel -C(=N-O-R₂)- verbunden ist, eine Verbindung der Formel VIII, worin n für 0 oder 1 steht, X Wasserstoff, Halogen oder Hydroxy bedeutet und R1die unter Formel I genannten Bedeutungen hat und worin die 4-Carboxylgruppe sowie weitere funktionelle Gruppen gegebenenfalls geschützt sind, mit einem Salz der Rhodanwasserstoffsäure, bzw. mit einem Isorhodanwasserstoffsäureester, oder, falls X₁ Wasserstoff bedeutet, mit Dirhodan behandelt,-oder
f) eine Verbindung der Formel IX, worin n für 0 oder 1 steht, Am, T und R₂ die unter Formel I genannten Bedeutungen haben und X₂ einen durch nucleophile Substitution ersetzbaren Rest darstellt und worin die 4-Carboxylgruppe sowie weitere funktionelle Gruppen gegebenenfalls geschützt sind, mit einer dem Rest R₋ entsprechenden organischen tertiären Base umsetzt oder
g) zur Herstellung von Verbindungen der Formel I, worin Am eine primäre, sekundäre oder tertiäre Aminogruppe ist, eine Verbindung der Formel X, worin n für 0 oder 1 steht, T, R₁ und R₂ die unter Formel I genannten Bedeutungen haben und Y Halogen bedeutet, mit Ammoniak oder einem primären oder sekundären Amin Am-H oder einem Metallamid davon umsetzt und, wenn erwünscht, eine erhältliche Verbindung der Formel I in eine andere Verbindung der Formel I umwandelt und/oder, wenn erwünscht, eine erhältliche Verbindung der Formel I, worin n 0 bedeutet, in eine Verbindung der Formel I überführt, worin n 1 bedeutet, und/oder eine Verbindung der Formel I, worin n 1 bedeutet, in eine Verbindung der Formel I überführt, worin n 0 bedeutet, und/oder in einer erhätlichen Verbindung der Formel I in geschützter Form vorliegende funktionelle Gruppen in die freien funktionellen Gruppen überführt und/oder ein erhältliches Salz in die freie Verbindung oder in ein anderes Salz überführt, und/oder eine erhältliche freie Verbindung der Formel I mit einer salzbildenden Gruppe von isomeren Verbindungen der Formel I in die einzelnen Isomeren auftrennt.

25. Die nach dem Verfahren gemäss Anspruch 24 herstellbaren Verbindungen.

## Patentansprüche (Patentansprüche für folgende(n) Vertragsstaat(en) : AT)

1. Verfahren zur Herstellung von 7ß-Aminothiadiazolylacetylamino-3- ammoniomethyl-3-cephem-4-carbonsäureverbindungen der Formel 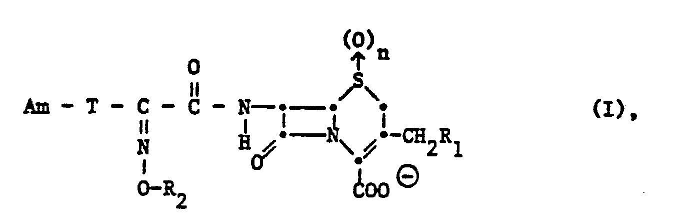 worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)- verbunden ist, R₁ eine unsubstituierte oder substituierte Pyrazoliogruppe, eine unsubstituierte oder substituierte Triazoliogruppe, eine Triniederalkylammoniogruppe oder eine Pyridiniogruppe der Formel 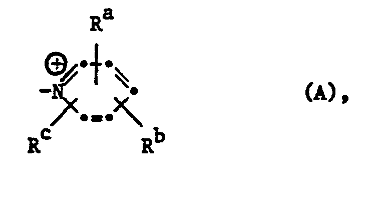 worin R durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl, bedeutet, R^{b} Carbamoyl oder Wasserstoff bedeutet oder die Bedeutungen von R^{a} hat und R^{c} Wasserstoff bedeutet oder die Bedeutungen von R hat, und R₂ Wasserstoff, unsubstituiertes oder substituiertes Niederalkyl oder Cycloalkyl oder unsubstituiertes oder substituiertes Carbamoyl bedeuten, und Salze von solchen Verbindungen, die eine salzbildende Gruppe aufweisen, dadurch gekennzeichnet, dass man
a) in einer Verbindung der Formel 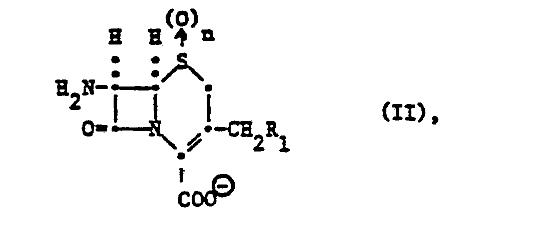 worin der Index n für 0 oder 1 steht, R₁ die unter Formel I genannten Bedeutungen hat, die 7ß-Aminogruppe gegebenenfalls durch eine die Acylierungsreaktion erlaubende Gruppe geschützt ist und die 4-Carboxygruppe gegebenenfalls geschützt ist, die 7ß-Aminogruppe durch Umsetzung mit einem den Acylrest einer Carbonsäure der Formel 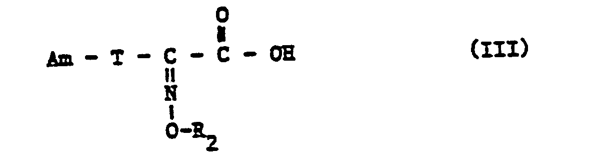 einführenden Acylierungsmittel, worin Am, T und R₂ die unter Formel I genannten Bedeutungen haben und vorhandene funktionelle Gruppen gegebenenfalls geschützt sind, acyliert oder
b) zur Herstellung einer Verbindung der Formel I, worin n für 0 steht, eine 2-Cephemverbindung der Formel 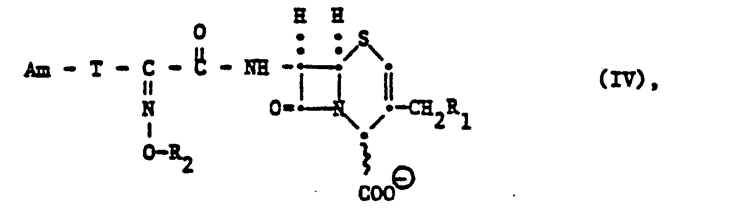 worin Am, T, R₁ und R₂ die unter Formel I genannten Bedeutungen haben und worin die 4-Carboxylgruppe sowie weitere funktionelle Gruppen gegebenenfalls geschützt sind, zur entsprechenden 3-Cephemverbindung isomerisiert oder
c) eine Verbindung der Formel 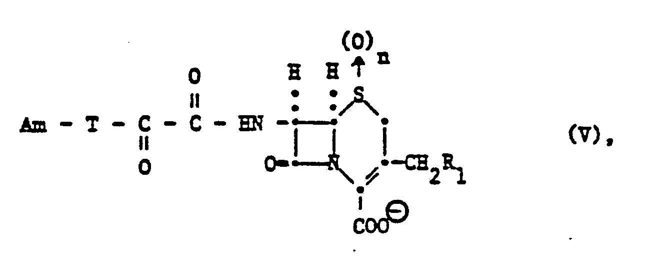 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Hydroxylaminderivat der Formel H₂N-O-R₂ (VI), worin R₂ die unter Formel I genannten Bedeutungen hat und funktionelle Gruppen gegebenenfalls geschützt sind, umsetzt oder
d) zur Herstellung einer Verbindung der Formel I, worin R₂ Wasserstoff bedeutet, eine Verbindung der Formel 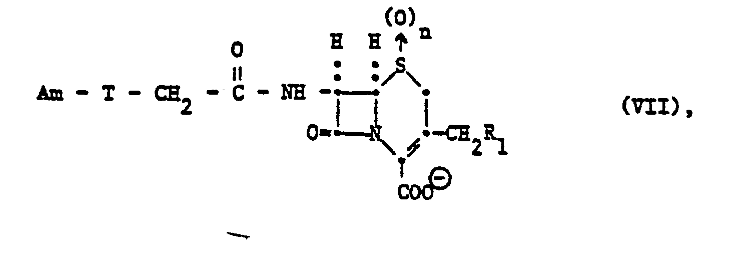 worin n für 0 oder 1 steht, Am, T und R₁ die unter Formel I genannten Bedeutungen haben und worin die 4-Carboxylgruppe sowie weitere funktionelle Gruppen gegebenenfalls geschützt sind, mit einem Nitrosierungsmittel behandelt oder
e) zur Herstellung einer Verbindung der Formel I, worin Am eine freie oder eine sekundäre Aminogruppe, T einen 1,2,4-Thiadiazolrest darstellt, der in 5-Stellung mit Am und in 3-Stellung mit der Gruppe der Formel -C(=N-O-R₂)- verbunden ist, eine Verbindung der Formel 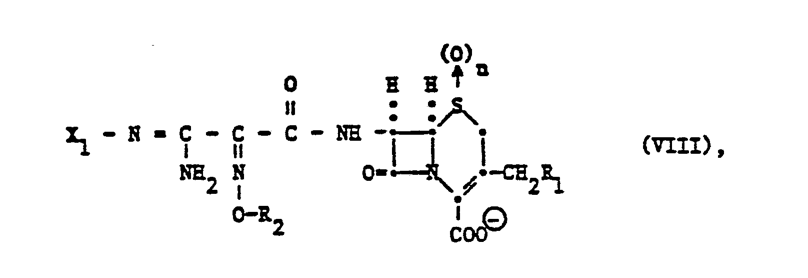 worin n für 0 oder 1 steht, X₁ Wasserstoff, Halogen oder Hydroxy bedeutet und R₁ die unter Formel I genannten Bedeutungen hat und worin die 4-Carboxylgruppe, sowie weitere funktionelle Gruppen gegebenenfalls geschützt sind, mit einem Salz der Rhodanwasserstoffsäure, bzw. mit einem Isorohodanwasserstoffsäureester oder, falls X₁ Wasserstoff bedeutet, mit Dirhodan behandelt oder
f) eine Verbindung der Formel 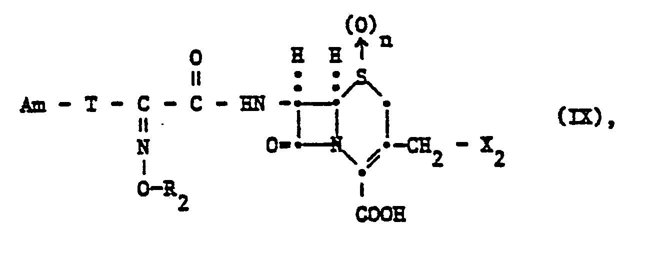 worin n für 0 oder 1 steht, Am, T und R₂ die unter Formel I genannten Bedeutungen haben und X₂ einen durch nucleophile Substitution ersetzbaren Rest darstellt und worin die 4-Carboxylgruppe sowie weitere funktionelle Gruppen gegebenenfalls geschützt sind, mit einer den Rest R₁ entsprechenden organischen tertiären Base umsetzt oder
g) zur Herstellung von Verbindungen der Formel I, worin Am eine primäre, sekundäre oder tertiäre Aminogruppe ist, eine Verbindung der Formel 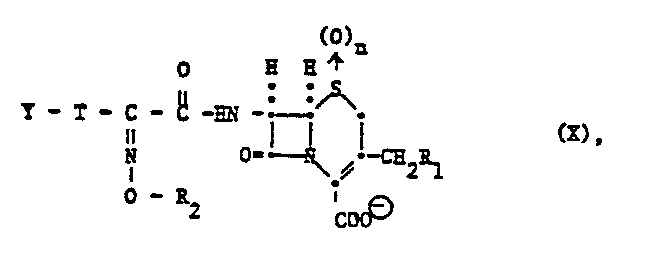 worin n für 0 oder 1 steht, T, R₁ und R₂ die unter Formel I genannten Bedeutungen haben und Y Halogen bedeutet, mit Ammoniak oder einem primären oder sekundären Amin Am-H oder einem Metallamid davon umsetzt und, wenn erwünscht, eine erhältliche Verbindung der Formel I in eine andere Verbindung der Formel I umwandelt und/oder, wenn erwünscht, eine erhältliche Verbindung der Formel I, worin n 0 bedeutet, in eine Verbindung der Formel I überführt, worin n 1 bedeutet, und/oder eine Verbindung der Formel I, worin n 1 bedeutet, in eine Verbindung der Formel I überführt, worin n 0 bedeutet, und/oder in einer erhältlichen Verbindung der Formel I in geschützter Form vorliegende funktionelle Gruppen in die freien funktionellen Gruppen überführt und/oder ein erhältliches Salz in die freie Verbindung der Formel I oder in ein anderes Salz überführt, und/oder eine erhältliche freie Verbindung der Formel I mit einer salzbildenden Gruppe in ein Salz überführt und/oder ein erhältliches Gemisch von isomeren Verbindungen in die einzelnen Isomeren auftrennt.

2. Verfahren zur Herstellung von Verbindungen der Formel I, worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)-verbunden ist, R₁ eine 2-Niederalkyl-l-pyrazoliogruppe, eine 3-Niederalkyl-l-triazoliogruppe, eine Triniederalkylammoniogruppe oder eine Pyridiniogruppe der Formel A, worin R durch Cycloalkyl, Phenyl, Hydroxy, Halogen, Cyano, Carbamoyl, Carboxyl oder Sulfo substituiertes Niederalkyl, unsubstituiertes oder durch Carboxy substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl bedeutet, R Carbamoyl oder Wasserstoff bedeutet oder die Bedeutungen von R^{a} hat und R^{c} Wasserstoff bedeutet oder die Bedeutungen von R^{a} hat, und R₂ Wasserstoff, unsubstituiertes oder substituiertes Niederalkyl oder Cycloalkyl oder unsubstituiertes oder substituiertes Carbamoyl bedeuten, und Salzen von solchen Verbindungen, die eine salzbildende Gruppe aufweisen.

3. Verfahren zur Herstellung von Verbindungen der Formel I, worin n für 0 oder 1 steht, Am unsubstituiertes oder substituiertes Amino, T einen Thiadiazolrest, welcher an einem Kohlenstoffatom mit Am und am anderen Kohlenstoffatom mit der Gruppe der Formel -C(=N-O-R₂)-verbunden ist, R₁ eine 2-Niederalkyl-l-pyrazoliogruppe, eine 3-Niederalkyl-l-triazoliogruppe, eine Triniederalkylammoniogruppe oder eine Pyridiniogruppe der Formel A, worin R^{a} durch Cycloalkyl, Phenyl, Hydroxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro, oder Hydroxysulfoniederalkyl bedeutet, R^{b} Carbamoyl oder Wasserstoff bedeutet oder die Bedeutungen von R^{a} hat und R^{c} Wasserstoff bedeutet oder die Bedeutungen von R^{a} hat, und R₂ Wasserstoff, unsubstituiertes oder substituiertes Niederalkyl oder Cycloalkyl oder unsubstituiertes oder substituiertes Carbamoyl bedeuten, und Salzen von solchen Verbindungen, die eine salzbildende Gruppe aufweisen.

4. Verfahren nach Anspruch 1 zur Herstellung von Verbindungen der Formel I, worin n für 0 steht, Am Amino, Niederalkylamino, Diniederalkylamino oder geschütztes Amino, T einen Thiadiazolrest, welcher an einem Kohlenstoffatom mit Am und am anderen Kohlenstoffatom mit der Gruppe der Formel -C(=N-O-R₂)- verbunden ist, R₁ 2-Niederalkyl-l-pyrazolio, 2-Carboxyniederalkyl-l-pyrazolio, 1-Carboxyniederalkyl-1-pyrazolio, 2-Niederalkoxycarbonylniederalkyl-l-pyrazolio, 3-Niederalkyl-1-triazolio oder eine Pyridiniogruppe der Formel A, worin R^{a} durch Cycloalkyl, Phenyl, Hydroxy, Niederalkoxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Thiocarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro oder Hydroxysulfoniederalkyl bedeutet, R^{b} Carbamoyl oder Wasserstoff bedeutet oder die Bedeutungen von R^{a} hat und R Wasserstoff bedeutet,und R₂ Wasserstoff, unsubstituiertes oder substituiertes Niederalkyl- oder Cycloalkyl oder unsubstituiertes oder substituiertes Carbamoyl bedeuten, wobei die Gruppe der Formel =N-O-R₂ insbesondere die syn(oder Z)-Form aufweist , sowie Salzen, insbesondere pharmazeutisch verwendbaren Salzen von solchen Verbindungen der Formel I, die salzbildende Gruppen aufweisen.

5. Verfahren nach Anspruch 3 zur Herstellung von Verbindungen der Formel I, worin n für 0 steht, Am Amino, Niederalkylamino, Diniederalkylamino oder geschütztes Amino, T einen Thiadiazolrest, welcher an einem Kohlenstoffatom mit Am und am anderen Kohlenstoffatom mit der Gruppe der Formel -C(=N-0-R₂)- verbunden ist, R₁ 2-Niederalkyl-l-pyrazolio, 3-Niederalkyl-l-triazolio oder eine Pyridiniogruppe der Formel A, worin R^{a} durch Cycloalkyl, Phenyl, Hydroxy, Halogen, Cyano, Carbamoyl, Carboxyl oder Sulfo substituiertes Niederalkyl, unsubstituiertes oder durch Carboxyl substituiertes Niederalkenyl oder Niederalkylthio, unsubstituiertes oder durch Niederalkyl, Niederalkanoyl oder Aminobenzolsulfonyl monosubstituiertes Amino, Diniederalkylamino, durch Niederalkyl, Hydroxyniederalkyl, Niederalkoxy, Hydroxy oder Cyano monosubstituiertes Carbamoyl, Diniederalkylcarbamoyl, Cycloalkyl, Phenyl, Hydroxy, Niederalkoxy, Halogen, Niederalkoxycarbonyl, Niederalkanoyloxy, Niederalkanoyl, Carboxyl, Sulfo, Cyano, Nitro oder Hydroxysulfoniederalkyl, R Carbamoyl oder Wasserstoff bedeutet oder die Bedeutungen von R hat und R^{c} Wasserstoff bedeutet, und R₂ Wasserstoff, unsubstituiertes oder substituiertes Niederalkyl- oder Cycloalkyl oder unsubstituiertes oder substituiertes Carbamoyl bedeutet, wobei die Gruppe der Formel =N-O-R₂ insbesondere die syn(oder Z)-Form aufweist, sowie Salzen, insbesondere pharmazeutisch verwendbaren Salzen von solchen Verbindungen der Formel I, die salzbildende Gruppen aufweisen.

6. Verfahren nach Anspruch 4 zur Herstellung von Verbindungen der Formel I, worin n für 0 steht, Am Amino, Niederalkylamino, z.B. Methylamino, oder geschütztes Amino, T einen Thiadiazolrest, welcher an einem Kohlenstoffatom mit Am und am anderen Kohlenstoffatom mit der Gruppe der Formel -C(=N-O-R₂)- verbunden ist, R₁ 2-Niederalkyl-1-pyrazolio, z.B. 2-Methyl-l-pyrazolio, 2-Carboxyniederalkyl-l-pyrazolio, z.B. 2-Carboxymethyl-l-pyrazolio, 1-Carboxyniederalkyl-1-pyrazolio, z.B. 1-Carboxymethyl-l-pyrazolio, 2-Niederalkoxycarbonylniederalkyl-l-pyrazolio, z.B. 2-Methoxycarbonylmethyl-l-pyrazolio, 3-Niederalkyl-l-triazolio, z.B. 3-Methyl-l-triazolio oder eine Pyridiniogruppe der Formel A, beispielsweise Hydroxyniederalkylpyridinio, z.B. 3- oder 4-Hydroxymethylpyridinio, Niederalkoxyniederalkylpyridinio, z.B. 4-Methoxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfo- äthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanohalogenmethylpyridinio, z.B. 3-Cyano-4-trifluormethylpyridinio oder Aminocarboxypyridinio, z.B. 2-Amino-3-carboxypyridinio,und R₂ Wasserstoff, Niederalkyl, z.B. Methyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl,bedeuten, wobei die Gruppe der Formel =N-0-R₂ die syn(oder Z)-Form aufweist, sowie Salzen, insbesondere pharmazeutisch verwendbaren Salzen von solchen Verbindungen der Formel I, die salzbildende Gruppen aufweisen.

7. Verfahren nach Anspruch 5 zur Herstellung von Verbindungen der Formel I, worin n für 0 steht, Am Amino, Niederalkylamino, z.B. Methylamino, oder geschütztes Amino, T einen Thiadiazolrest, welcher an einem Kohlenstoffatom mit Am und am anderen Kohlenstoffatom mit Am und am anderen Kohlenstoffatom mit der Gruppe der Formel -C(=N-O-R₂)- verbunden ist, R₁ 2-Niederalkyl-l-pyrazolio, z.B. 2-Methyl-l-pyrazolio, 3-Niederalkyl-l-triazolio, z.B. 3-Methyl-l-triazolio oder eine Pyridiniogruppe der Formel A, beispielsweise Hydroxyniederalkylpyridinio, z.B. 3- oder 4-Hydroxymethylpyridinio, Cyanoniederalkylpyridinio, z.B. 3-Cyanomethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Halogenpyridinio, z.B. 3-Brom- oder 4-Brompyridinio, Carboxypyridinio, z.B. 4-Carboxypyridinio, Sulfopyridinio, z.B. 3-Sulfopyridinio, Cyanpyridinio, z.B. 3-Cyanpyridinio, Carboxyniederalkylcarbamoylpyridinio, z.B. 3-Carboxymethyl-4-carba- moylpyridinio, Aminocarbamoylpyridinio, z.B. 2-Amino-5-carbamoyl- pyridinio, Carboxycarbamoylpyridinio, z.B. 3-Carboxy-4-earbamoyl- pyridinio, Cyanohalogenmethylpyridinio, z.B. 3-Cyano-4-trifluormethyl- pyridinio oder Aminocarboxypyridinio, z.B. 2-Amino-3-carboxypyridinio und R₂ Wasserstoff, Niederalkyl, z.B. Methyl, Carboxyniederalkyl, z.B. 2-Carboxy-2-propyl, Carbamoyl, Niederalkylcarbamoyl, z.B. Methylcarbamoyl, oder Aminoniederalkyl, z.B. 2-Aminoäthyl, bedeuten, wobei die Gruppe der Formel =N-0-R₂ die syn(oder Z)-Form aufweist, sowie Salzen, insbesondere pharmazeutisch verwendbaren Salzen von solchen Verbindungen der Formel I, die salzbildende Gruppen aufweisen.

8. Verfahren nach Anspruch 6 zur Herstellung von Verbindungen der Formel I, worin n für 0 steht, Am Amino, T einen 1.,2,4-Thiadiazolrest, welcher in 5-Stellung mit Am und in 3-Stellung mit der Gruppe der Formel -C(=_{N}-₀-_{R2})- verbunden ist, R₁ 2-Niederalkyl-l-pyrazolio, z.B. 2-Methyl-l-pyrazolio, 2-Carboxyniederalkyl-l-pyrazolio, z.B. 2-Carboxymethyl-1-pyrazolio, 2-Niederalkoxycarbonylniederalkyl-l-pyrazolio, z.B. 2-Methoxycarbonylmethyl-l-pyrazolio, 3-Niederalkyl-l-triazolio, z.B. 3-Methyl-l-triazolio, oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Sulfoniederalkylpyridinio, z.B. 4-(2-Sulfoäthylpyridinio), Halogenpyridinio, z.B. 3-Brompyridinio, Carboxypyridinio, z.B. 4-Carboxypyridinio, Sulfopyridinio, z.B. 3-Sulfopyridinio oder Aminocarbamoylpyridinio, z.B. 2-Amino-5-carbamoylpyridinio, und R₂ Wasserstoff, Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel =N-0-R₂ die syn(oder Z)-Form aufweist, sowie Salzen, in erster Linie pharmazeutisch verwendbaren Salzen von Verbindungen der Formel I.

9. Verfahren nach Anspruch 7 zur Herstellung von Verbindungen der Formel I, worin n für 0 steht, Am_{.}Amino, T einen 1,2,4-Thiadiazolrest, welcher in 5-Stellung mit Am und in 3-Stellung mit der Gruppe der Formel -C(=N-O-R₂)- verbunden ist, R₁ 2-Niederalkyl-l-pyrazolio, z.B. 2-Methyl-l-pyrazolio, 3-Niederalkyl-l-triazolio, z.B. 3-Methyl-l-triazolio, oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Sulfoniederalkylpyridinio, z.B. 4-(2-Sulfoäthylpyridinio), Halogenpyridinio, z.B. 3-Brompyridinio, Carboxypyridinio, z.B. 4-Carboxypyridinio, Sulfopyridinio, z.B. 3-Sulfopyridinio oder Aminocarbamoylpyridinio, z.B. 2-Amino-5-carbamoylpyridinio, und R₂ Wasserstoff, Niederalkyl, z.B. Methyl, Carboxyniederalkyl, z.B. 2-Carboxy-2-propyl, Carbamoyl oder Niederalkylcarbamoyl, z.B. Methylcarbamoyl, bedeuten, wobei die Gruppe der Formel =N-0-R₂ die syn(oder Z)-Form aufweist, sowie Salzen, in erster Linie pharmazeutisch verwendbaren Salzen von Verbindungen der Formel I.

10. Verfahren gemäss Anspruch 9 zur Herstellung von Verbindungen der Formel I,worin n für 0 steht, Am Amino, T einen 1,2,4-Thiadiazolrest, welcher in 5-Stellung mit Am und in 3-Stellung mit der Gruppe der Formel -C(N-O-R₂)- verbunden ist, R₁ 2-Niederalkyl-1-pyrazolio, z.B. 2-Methyl-l-pyrazolio oder eine Pyridiniogruppe der Formel A, beispielsweise Hydroxyniederalkylpyridinio, z.B. 4-Hydroxymethylpyridinio, Carboxyniederalkylpyridinio, z.B. 3-Carboxymethylpyridinio, Halogenpyridinio, z.B. 3-Brompyridinio, oder Carboxypyridinio, z.B. 4-Carboxypyridinio, und R₂ Niederalkyl, z.B. Methyl oder Carboxyniederalkyl, z.B. 2-Carboxy-2-propyl oder Aminocarbamoylpyridinio, z.B. 2-Amino-5-carbamoylpyridinio,bedeuten, wobei die Gruppe der Formel =N-O-R₂ die syn(oder Z)-Form aufweist, sowie Salzen, in erster Linie pharmazeutisch verwendbaren Salzen von Verbindungen der Formel I.

11. Verfahren zur Herstellung von 7ß-[2-(5-Amino-l,2,4-thiadiazol-3-yl)-2-Z-methoxyiminoacetamido]-3-(4-carboxypyridiniomethyl)-3-cephem-4-carboxylat, gemäss Anspruch 10.

12. Verfahren zur Herstellung von 7ß-[2-(5-Amino-l,2,4-thiadiazol-3-yl)-2-Z-2-carboxyprop-2-yloxyiminoacetamido]-3-(3-carboxymethyl- pyridiniomethyl)-3-cephem-4-carboxylat, gemäss Anspruch 10.

13. Verfahren zur Herstellung von 7ß-[2-(5-Amino-l,2,4-thiadiazol-3-yl)-2-Z-methoxyiminoacetamido]-3-(2-methyl-l-pyrazoliomethyl)-3-cephem-4-carboxylat, gemäss Anspruch 10.

14. Verfahren zur Herstellung von 7ß-[2-(5-Amino-1,2,4-thiadiazol-3-yl)-2-Z-methoxyiminoacetamido]-3-[4-(2-sulfoäthyl)-pyridiniomethyl]-3-cephem-4-carboxylat, gemäss Anspruch 10.

15. Verfahren zur Herstellung von 7ß-[2-(5-Amino-l,2,4-thiadiazol-3-yl)-2-Z-methoxyiminoacetamido]-3-(2-amino-5-carbamoylpyridiniomethyl)-3-cephem-4-earboxylat, gemäss Anspruch 10.

16. Verfahren zur Herstellung von 7ß-[2-(5-Amino-l,2,4-thiadiazol-3-yl)-2-Z-methoxyiminoacetamido]-3-(3-brompyridiniomethyl)-3-cephem-4-carboxylat, gemäss Anspruch 10.

17. Verfahren zur Herstellung von 7β-[2-(5-Amino-1,2,4-thiadiazol-3-yl)-2-Z-methoxyiminoacetamido]-3-(4-hydroxymethylpyridiniomethyl)-3-cephem-4-carboxylat, gemäss Anspruch 10.
